# EUROPEAN PATENT SPECIFICATION

(11) **EP 4 269 885 B1**
(45) Date of publication and mention of the grant of the patent: **21.01.2026**
(21) Application number: 23156516.9
(22) Date of filing: 14.02.2023
(51) Int. Cl.: F24F 1/0076, F24F 3/16, F24F 8/30, F24F 11/30, F24F 11/65, F24F 1/0038, F24F 3/00, F24F 110/50, F24F 110/64

(54) **AIR CONDITIONER COMPRISING A ION GENERATING DEVICE**
KLIMAANLAGE MIT EINER IONENERZEUGUNGSVORRICHTUNG
CLIMATISEUR COMPRENANT UN DISPOSITIF DE GÉNÉRATION D'IONS

(30) Priority: 27.04.2022 KR 20220051844
(43) Date of publication of application: 01.11.2023
(73) Proprietor: LG Electronics Inc., Seoul 07336 (KR)
(72) Inventor: Son, Ilna, 08592 Seoul (KR); Kim, Bongjun, 08592 Seoul (KR); Jeong, Minwoo, 08592 Seoul (KR); Kim, Sehyoun, 08592 Seoul (KR)
(74) Representative: Ter Meer Steinmeister & Partner

(56) References cited:
- EP-A1- 4 180 736
- CN-U- 206 959 157
- CN-U- 206 959 160
- KR-B1- 101 427 848

## Description

This application claims priority of Korean Patent Application No. 10-2022-0051844, filed on April 27, 2022.

### BACKGROUND

### 1. Field of the invention

The present invention relates to an air conditioner. More particularly, the present invention relates to an air conditioner having an ion generating device.

### 2. Description of the Related Art

In general, an air conditioner refers to a device that heats and cools a room through compression, condensation, expansion, and evaporation of a refrigerant. Such an air conditioner can improve the air quality of room by exchanging outdoor air with a room air through a ventilation device. In addition, the ventilation device may increase the temperature of the air supplied to the room by using a high-temperature combustion gas of a gas furnace.

Such an air conditioner may include an ion generating device to remove bacteria or microorganisms living in the ventilation device. For example, the ion generating device generates negative ions or positive ions by applying a pulsed high voltage to a discharge electrode. The electric field formed by the high voltage applied to the discharge electrode accelerates free electrons in the surrounding air, and the accelerated free electrons collide with neutral molecules in the air, such as nitrogen or oxygen, to ionize the neutral molecules. The negative ions or positive ions generated by the ion generating device provide beneficial effects such as deodorization, as well as sterilization.

KR 10-0762142 (Registration date: September 20, 2007) discloses an air conditioner that removes bacteria or microorganisms living in the inside of a duct through a sterilization kit. Specifically, the sterilization kit of the above air conditioner removes bacteria or microorganisms that exist in the air or live in the inside of the duct by spraying a sterilizing solution into the air supplied from the outside to the room.

However, the sterilization kit of the above air conditioner has the inconvenience of having to periodically refill the sterilizing solution. In addition, the sterilizing solution of the sterilization kit is loaded in the airflow of a blower operated for air conditioning in the room and provided to a duct, or the like. That is, there is a problem in that the sterilization kit can be operated only while the air conditioning operation is being performed, and it cannot prevent the propagation of bacteria or microorganisms while the air conditioning operation is stopped. In other words, when the air conditioner is operated after not operating for a long time, the polluted air or material remaining in the duct is supplied to the room, which may cause discomfort to occupants and may adversely affect the room air.

KR 10-2009-0084429 (Publication date: August 5, 2009) discloses a vehicle air conditioner having an ion generating device. However, the ion generating device of the vehicle air conditioner operates and provides ions to the occupant, only while a blower for vehicle air conditioning is operating. That is, similar to the above-mentioned registered patent, the above vehicle air conditioner also has a problem in that it cannot prevent the propagation of bacteria or microorganisms inside the duct in which the ion generating device is installed while the vehicle air conditioning operation is stopped.

EP 4 180 736 A1, an Art 54(3) EPC document, discloses an ion generating device and an air conditioner having the same. The air conditioner may include a housing; a blower which causes a flow of air passing through an inner space of the housing; a heat exchanger located in the inner space of the housing; and an ion generating device which is spaced apart from the heat exchanger, and coupled to an inner side of the housing, wherein the ion generating device may include: a hollow body; a fan which is coupled to one side of the body, and causes a flow of air passing through an inside of the body; and an ionizer which is coupled to the other side of the body, and generates ion, wherein the ionizer may include a case hole which is formed in a portion of the ionizer facing the inside of the body, and communicates with the inside of the body. CN 206 959 157 U describes a further air conditioner comprising a ion generating device installed in a housing comprising a fan.

### SUMMARY

An object of the present invention is to solve the above and other problems. Another object may be to provide an air conditioner capable of heating or cooling outdoor air and supplying it to a room.

Another object may be to provide an ion generating device capable of removing bacteria or microorganisms that grow in an environment inside an air conditioner, that is, in an environment which is repeatedly exposed to low temperature and high humidity according to changes in temperature and humidity, and in which condensate can be generated.

Another object may be to provide an ion generating device that can be operated continuously for a long time and is easy to maintain, manage and repair.

Another object may be to provide an ion generating device that has a fan and provides ions to a disinfection target space as a whole.

Another object may be to provide an ion generating device that has a fan and can be operated while the air conditioning operation is stopped, and a method for disinfection operation.

Another object may be to provide a method for stepwise controlling the operating time of an ion generating device, based on the air conditioning operating time and/or fine dust information in the air during a disinfection operation.

Another object may be to provide a coupling structure and an optimal installation location of a ventilation device and an ion generating device of the air conditioner.

Another object may be to provide an ion generating device capable of minimizing air flow resistance during an air conditioning operation.

The object is solved by the invention set out by the features of the independent claim. Preferred embodiments are given in the dependent claims.

An aspect of the present invention provides an air conditioner according to claim 1. Further preferred aspects of the present invention are provided in the dependent claims.

### BRIEF DESCRIPTION OF THE DRAWINGS

The above and other objects, features and advantages of the present invention will be more apparent from the following detailed description in conjunction with the accompanying drawings, in which:
FIG. 1 and 2 are views for explaining a configuration of an air conditioner according to an embodiment of the present invention;
FIG. 3 is a view for explaining a gas furnace of an air conditioner according to an embodiment of the present invention;
FIG. 4 is a perspective view of an ion generating device of an air conditioner according to an embodiment of the present invention;
FIGS. 5 and 6 are views for explaining an ionizer of an ion generating device according to an embodiment of the present invention;
FIGS. 7 and 8 are views for explaining an ion generator of an ionizer according to an example of the present invention;
FIGS. 9 and 10 are views for explaining an ion generator of an ionizer according to another example of the present invention;
FIG. 11 is a cross-sectional view of an ion generator according to an embodiment of the present disclosure;
FIG. 12 is a view for explaining a fan of an ion generating device according to an embodiment of the present invention;
FIG. 13 is a perspective cutaway view of an ion generating device according to an embodiment of the present invention;
FIG. 14 is a view for explaining an ion generating device installed in a first space of an air conditioner according to an embodiment of the present invention;
FIG. 15 is a view for explaining an ion generating device installed in a second space of an air conditioner according to an embodiment of the present invention;
FIG. 16 is a graph showing a change in the amount of ions according to a distance between a fan and a housing of an ion generating device according to an embodiment of the present invention;
FIGS. 17 and 18 are views for explaining an optimal location of an ion generating device according to an embodiment of the present invention;
FIG. 19 is a control configuration diagram of an air conditioner according to an embodiment of the present invention;
FIG. 20 is a flowchart illustrating a method for controlling an air conditioning operation and a disinfection operation according to an embodiment of the present invention;
FIG. 21 is a view for explaining a difference in ion distribution depending on an operation of a blower in a disinfection operation according to an embodiment of the present invention;
FIG. 22 is a flowchart illustrating a method for controlling a disinfection operation according to an embodiment of the present invention;
FIG. 23 is a control configuration diagram of an air conditioner according to an embodiment of the present invention;
FIG. 24 is a flowchart illustrating a method for controlling an air conditioning operation and a disinfection operation according to an embodiment of the present invention; and
FIGS. 25 and 26 are views for comparing and explaining the removal rates of microorganisms when an ion generating device is operated to perform a disinfection operation for a ventilation device, and when a ventilation device is left unattended, after an air conditioning operation is completed.

### DETAILED DESCRTPTION OF THE PREFERRED EMBODIMENTS

Description will now be given in detail according to embodiments disclosed herein, with reference to the accompanying drawings. For the sake of brief description with reference to the drawings, the same or equivalent components may be denoted by the same reference numbers, and description thereof will not be repeated.

In general, suffixes such as "module" **and** "unit" may be used to refer to elements or components. Use of such suffixes herein is merely intended to facilitate description of the specification, and the suffixes do not have any special meaning or function.

In the present disclosure, that which is well known to one of ordinary skill in the relevant art has generally been omitted for the sake of brevity. The accompanying drawings are used to assist in easy understanding of various technical features and it should be understood that the embodiments presented herein are not limited by the accompanying drawings.

It will be understood that although the terms first, second, etc. may be used herein to describe various elements, these elements should not be limited by these terms. These terms are only used to distinguish one element from another.

It will be understood that when an element is referred to as being "connected with" another element, there may be intervening elements present. In contrast, it will be understood that when an element is referred to as being "directly connected with" another element, there are no intervening elements present.

A singular representation may include a plural representation unless context clearly indicates otherwise.

In the following description, even if the embodiment is described with reference to specific drawings, if necessary, reference numerals not appearing in the specific drawings may be referred to, and reference numerals not appearing in the specific drawings are used in a case where the above reference numerals appear in the other figures.

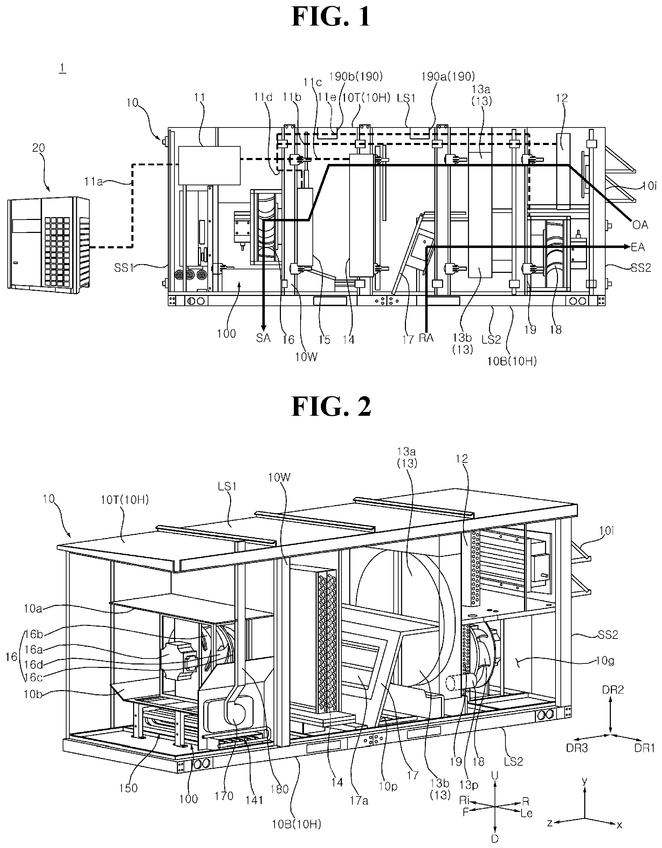

The direction indications of up(U, y), down(D), left(Le, x), right(Ri), front(F, z), and rear(R) indicated in FIG. 2 and the like are for convenience of explanation, and the technical concept of the present invention is not limited thereto.

Referring to FIGS. 1 and 2, an air conditioner 1 may include an outdoor unit 20 and a ventilation device 10. The outdoor unit 20 may include a compressor (not shown) that compresses a refrigerant, and an outdoor heat exchanger (not shown) that heat-exchanges the refrigerant with outdoor air. The outdoor unit 20 may be connected to the ventilation device 10 through a first refrigerant pipe 11a. The refrigerant may circulate through the outdoor unit 20 and the ventilation device 10 through the refrigerant pipe.

A housing 10H may include a first long side LS1 and a second long side LS2 opposite to the first long side LS1. The first long side LS1 and the second long side LS2 may be collectively referred to as a long side LS1, LS2. The housing 10H may include a first short side SS1 adjacent to the long side LS1, LS2 and a second short side SS2 opposite to the first short side SS1. The first short side SS1 and the second short side SS2 may be collectively referred to as a short side SS1, SS2.

A direction perpendicular to the long side LS1, LS2 and the short side SS1, SS2 may be referred to as a first direction DR1 or a left-right direction. The direction parallel to the short side SS1, SS2 may be referred to as a second direction DR2 or an up-down direction. The direction parallel to the long side LS1, LS2 may be referred to as a third direction DR3 or a front-rear direction.

The side of the first long side LS1 may be referred to as an upper side (U, y), and the side of the second long side LS2 may be referred to as a lower side (D). The side of the first short side SS1 may be referred to as a front side (F, z), and the side of the second short side SS2 may be referred to as a rear side (R). In the first direction DR1, a direction toward one end of the short side SS1, SS2 may be referred to as a left side (Le, x), and a direction toward the other end of the short side SS1, SS2 may be referred to as a right side (Ri).

A portion forming the first long side LS1 of the housing 10H may be referred to as a top part 10T, and a portion forming the second long side LS2 of the housing 10H may be referred to as a bottom part 10B.

The ventilation device 10 may include a refrigerant distributor 11, a plurality of heat exchangers 12, 13, 14, 15, 19, a blower 16, a damper mount 17, and an exhaust fan 18. The refrigerant distributor 11, the plurality of heat exchangers 12, 13, 14, 15, 19, the blower 16, the damper mount 17, and the exhaust fan 18 may be installed inside the housing 10H.

A supply air passage OA-SA may be formed between a first inlet 10i and a first outlet (not shown). The first inlet 10i may be formed to penetrate the second short side SS2, and may be adjacent to the first long side LS1. The first outlet may be formed to penetrate the second long side LS2 and may be adjacent to the first short side SS1. Outdoor air OA may flow into the first inlet 10i, and the first inlet 10i may be referred to as an outdoor air inlet. Supply air (SA) may be supplied into a room through the first outlet, and the first outlet may be referred to as a supply air outlet.

The blower 16 may be located in the supply air passage OA-SA adjacent to the first outlet. The blower 16 may cause a flow of air along the supply air passage OA-SA. The blower 16 may be referred to as a supply air fan 16 or a plug fan. Meanwhile, a supply air duct (not shown) may be connected to the second long side LS2 and may communicate with the first outlet and an indoor space. For example, the air volume per minute of the blower 16 may be 3,000 to 5,000 cubic feet per minute (CFM).

An exhaust air passage RA-EA may be formed between a second inlet 10p and a second outlet 10g. The second inlet 10p may be formed to penetrate the second long side LS2 and may be spaced apart from the first outlet. The second outlet 10g may be formed to penetrate the second short side SS2 and may be adjacent to the second long side LS2. Room air (or return air, RA) may flow into the second inlet 10p, and the second inlet 10p may be referred to as a room air inlet. Exhaust air (EA) may be discharged to the outside through the second outlet 10g, and the second outlet 10g may be referred to as an exhaust air outlet.

The exhaust fan 18 may be located in the exhaust air passage RA-EA adjacent to the second discharge port 10g. The exhaust fan 18 may cause a flow of air along the exhaust air passage RA-EA. The exhaust fan 18 may be referred to as a blower or a plug fan. Meanwhile, a room air duct (not shown) may be connected to the second long side LS2, and may communicate with the second inlet 10p and an indoor space.

The damper mount 17 may partition an inner space of the housing 10H between a recovery wheel 13 described later and the heat exchanger 14 into a space that the supply air passage OA-SA forms, and a space that the exhaust air passage RA-SA forms. The damper mount 17 may be installed near the second inlet 10p of the housing 10H, and may include an inclination portion (unsigned) and a horizontal portion (unsigned). Accordingly, the supply air passage OA-SA may be located in the upper side of the damper mount 17, and the exhaust air passage RA-SA may be located in the lower side of the damper mount 17.

The damper 17a may be installed in the inclination portion of the damper mount 17. When the damper 17a is opened, the supply air passage OA-SA and the exhaust air passage RA-SA may communicate with each other. When the damper 17a is closed, the supply air passage OA-SA and the exhaust air passage RA-SA may be separated from each other. For example, in the initial stage of the heating operation of the air conditioner, the blower 16 may be operated but the exhaust fan 18 may be stopped, and the damper 17a may be opened.

The refrigerant distributor 11 may be adjacent to the first long side LS1 and the first short side SS1. One side of the refrigerant distributor 11 may be connected to the first refrigerant pipe 11a. The other side of the refrigerant distributor 11 may be connected to a plurality of refrigerant pipes 11b, 11c, 11d, 11e. For example, the refrigerant distributor 11 may open and close the passage of each refrigerant pipe through a solenoid valve. Here, each refrigerant pipe 11b, 11c, 11d, 11e may include a refrigerant pipe providing a passage of the refrigerant supplied to each heat exchanger 12, 14, 15, 19, and a refrigerant pipe providing a passage of the refrigerant that has passed through each heat exchanger 12, 14, 15, 19. In addition, each expansion valve (not shown) may be connected to each refrigerant pipe 11b, 11c, 11d, 11e, and may expand the refrigerant flowing through each refrigerant pipe 11b, 11c, 11d, 11e. For example, the expansion valve may be an electronic expansion valve (EEV) capable of adjusting the opening degree. In this case, when the expansion valve is fully opened, the expansion valve may not expand the refrigerant.

A radiator 12 may be located in the supply air passage OA-SA adjacent to the first inlet 10i. A high-temperature cooling water described later may pass through the radiator 12. Accordingly, the radiator 12 may heat the air flowed into the first inlet 10i. The radiator 12 may be referred to as a heat dissipation coil.

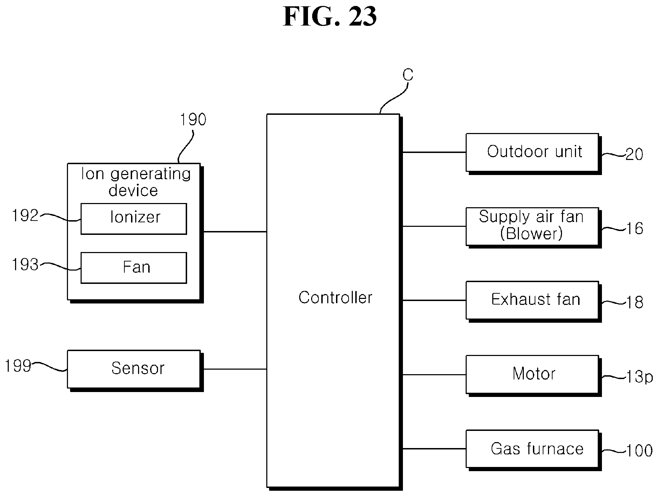

The heat exchanger 14 may be located downstream of the radiator 12 in the supply air passage OA-SA. The heat exchanger 14 may be vertically disposed within the housing 10H. The size of the heat exchanger 14 may be larger than the size of the radiator 12. The second refrigerant pipe 11c may provide a refrigerant passage connecting the refrigerant distributor 11 and the heat exchanger 14. The heat exchanger 14 may be referred to as a main heat exchanger or a cooling/heating coil. The heat exchanger 14 may be referred to as a second heat exchanger 14. Meanwhile, a filter 14a (see FIG. 23) may be located upstream of the heat exchanger 14.

A reheater 15 may be located downstream of the heat exchanger 14 in the supply air passage OA-SA. The reheater 15 may be vertically disposed within the housing 10H. The size of the reheater 15 may be smaller than the size of the heat exchanger 14. A third refrigerant pipe 11d may provide a refrigerant passage connecting the refrigerant distributor 11 and the reheater 15. The reheater 15 may be referred to as a reheat coil. The reheater 15 may be referred to as a third heat exchanger 15.

Meanwhile, the reheater 15 may be operated based on the indoor set temperature and set humidity. The reheater 15 may face the blower 16 with respect to a base 10W in which the reheater 15 is installed.

A recovery coil 19 may be located in the exhaust air passage RA-EA adjacent to the exhaust fan 18. The recovery coil 19 may be vertically disposed within the housing 10H. A fourth refrigerant pipe 11e may provide a refrigerant passage connecting the refrigerant distributor 11 and the recovery coil 19. Meanwhile, the heat transmission direction of the recovery coil 19 to the air may be opposite to the heat transmission direction of the heat exchanger 14 to the air.

A recovery wheel 13 may have a flat cylinder shape as a whole. A honeycomb structure may be formed inside the recovery wheel 13, and air may pass through the honeycomb structure. The recovery wheel 13 may be rotated by the power of the motor 13p. A rotation axis of the recovery wheel 13 may be a length direction axis of the recovery wheel 13, and the recovery wheel 13 may be rotated in a circumferential direction of the recovery wheel 13. For example, the power of the motor 13p may be transmitted to the recovery wheel 13 by using a belt and a pulley.

In addition, a first portion 13a of the recovery wheel 13 may be located in the supply air passage OA-SA. In the supply air passage OA-SA, the first portion 13a may be located between the radiator 12 and the heat exchanger 14. In addition, a second portion 13b of the recovery wheel 13 may be located in the exhaust air passage RA-EA. In the exhaust air passage RA-EA, the second portion 13b may be located between the inclination portion of the damper mount 17 and the recovery coil 19. In this case, a portion corresponding to the first portion 13a or the second portion 13b of the recovery wheel 13 may be changed in response to the rotation of the recovery wheel 13. The recovery wheel 13 may be referred to as a first heat exchanger 13.

Accordingly, the recovery wheel 13 may recover sensible heat and latent heat by using the temperature difference and humidity difference between the outdoor air OA and the room air RA. The recovery wheel 13 may be referred to as an energy recovery wheel (ERW).

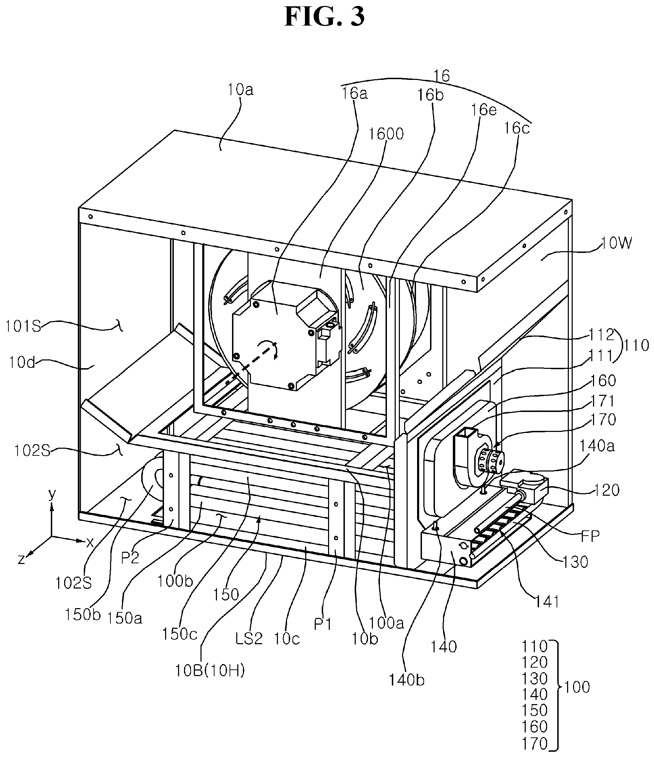

Referring to FIGS. 2 and 3, the blower 16 may include a motor 16a, a hub 16b, a shroud 16c, and a plurality of blades 16d. The hub 16b, the shroud 16c, and the plurality of blades 16d may be collectively referred to as an impeller.

The motor 16a may provide rotational force. The motor 16a may be a centrifugal fan motor. The motor 16a may form a front end of the blower 16, and the rotation shaft of the motor 16a may extend rearward from the motor 16a. The length direction of the rotation shaft of the motor 16a may be referred to as a shaft direction of the blower 16.

The hub 16b may be located in the rear side of the motor 16a and may be fixed to the rotation shaft of the motor 16a. The hub 16b may have a disk shape.

The shroud 16c may be located in the rear side of the hub 16b and may have a ring plate shape. The shroud 16c may be rotatably coupled to the base 10W. For example, the inlet (unsigned) may be fixed to the front surface of the base 10W between the shroud 16c and the base 10W, and may have a hyperbolic cylinder or funnel shape. In this case, the shroud 16c may be rotatably coupled to the inlet. The hole formed inside the shroud 16c, the inner space of the inlet, and the hole (not shown) formed in the base 10W may communicate with each other, and be located in the supply air passage OA-SA (see FIG. 1).

The plurality of blades 16d may be located between the inner circumference and the outer circumference of the ring-shaped shroud 16c. The plurality of blades 16d may be coupled to the hub 16b and the shroud 16c between the hub 16b and the shroud 16c. The plurality of blades 16d may be formed as one body with the shroud 16c and the hub 16b.

In addition, the plurality of blades 16d may be spaced apart from each other in the rotational direction of the rotation shaft of the motor 16a. Each of the plurality of blades 16d may be convexly curved in the rotational direction of the rotation shaft. For example, a blade located close to the mount plate 110 described later among the plurality of blades 16d may be convex toward the mount plate 110.

Accordingly, when the impeller 16a, 16b, 16c rotate clockwise according to the driving of the motor 16a, air may flow into in the axial direction of the blower 16 through the hole of the base 10W, and may be pressed by the plurality of blades 16d to be discharged in the radial direction of the blower 16).

The horizontal plate 10a may be vertically disposed on the front surface of the base 10W, and may be coupled to the front surface of the base 10W. The horizontal plate 10a may be located in the upper side of the blower 16. The horizontal plate 10a may be referred to as a first horizontal wall or a first panel. Meanwhile, the frame 16e may form a skeleton of the blower 16, and a motor mount 1600 on which the motor 16a is mounted may be coupled thereto. The frame 16e may be coupled to the lower side of the horizontal plate 10a.

A top plate 10b may be disposed perpendicular to the front surface of the base 10W, and may be coupled to the front surface of the base 10W. The top plate 10b may be located in the lower side of the blower 16. The top plate 10b may be referred to as a second horizontal wall or a second panel. A top hole 100a may be formed by penetrating the top plate 10b in the up-down direction. The top hole 100a may be formed to be long in the left-right direction. In the up-down direction, at least a portion of the top hole 100a may overlap the blower 16.

The bottom plate 10c may be vertically disposed on the front surface of the base 10W, and may be coupled to the front surface of the base 10W. The bottom plate 10c may face the horizontal plate 10a with respect to the top plate 10b. The bottom plate 10c may form a portion of the second long side LS2 of the housing 10H. The bottom hole 100b may be formed by penetrating the bottom plate 10c in the up-down direction. The bottom hole 100b may be formed to be long in the left-right direction. In the up-down direction, the bottom hole 100b may face the top hole 100a.

A side plate 10d may be disposed perpendicular to the front surface of the base 10W, and may be coupled to the front surface of the base 10W. The side plate 10d may be coupled to the right side of the horizontal plate 10a, the right side of the top plate 10b, and the right side of the bottom plate 10c.

The mount plate 110 may include a first plate 111 and a second plate 112. The first plate 111 may be disposed perpendicular to the front surface of the base 10W and the upper surface of the bottom plate 10c, and may be coupled to the front surface of the base 10W and the upper surface of the bottom plate 10c. The first plate 111 may be coupled to the left side of the top plate 10b. The second plate 112 may extend obliquely from the upper end of the first plate 111 in a direction away from the blower 16. In this case, the left side of the base 10W, the left side of the horizontal plate 10a, the left side of the second plate 112, and the left side of the bottom plate 10c may be connected to the left side portion of the housing 10H.

A first space 101S may be formed between the horizontal plate 10a and the top plate 10b. A vertical plate (not shown) may be connected to the front end of the horizontal plate 10a and the front end of the top plate 10b, and may close the front side of the first space 101S.

A second space 102S may be formed between the top plate 10b and the bottom plate 10c. The vertical plate may be connected to the front end of the top plate 10b and the front end of the bottom plate 10c, and may close the front side of the second space 102S. The second space 102S may communicate with the first space 101S through the top hole 100a, and may communicate with the indoor space through the bottom hole 100b.

Referring back to FIG. 3, a gas furnace 100 may include a fuel valve 120, a manifold 130, a burner 141, a heat exchanger 150, a collect box 160, and an inducer 170.

The fuel valve 120 may supply fuel from a fuel pipe (not shown) to the manifold 130, or may block the supply of the fuel to the manifold 130. For example, the fuel may be liquefied natural gas (LNG) or liquefied petroleum gas (LPG). Meanwhile, the amount of the fuel supplied to the manifold 130 may be modulated by adjusting the opening degree of the fuel valve 120. In other words, the thermal power of the gas furnace 100 may be modulated in stages by using the fuel valve 120. The fuel valve 120 may be referred to as a modulating valve.

The burner 141 may receive the fuel from the manifold 130. For example, primary air may flow into the burner 141 through a space between the burner 141 and the manifold 130. In this case, the fuel may pass through the burner 141 and be mixed with the primary air. The burner 141 may burn the fuel. When the fuel is burned, a flame and high-temperature combustion gas may be generated. For example, a plurality of burners 141 may be provided. The plurality of burners 141 may be installed inside the burner box 140. The burner box 140 may be installed in the left side of the first plate 111 of the mount plate 110.

For example, an igniter 140a may be adjacent to an exit port of a burner located in one end of the plurality of burners 141, and may burn fuel that has passed through the burner. In this case, the flame formed at the exit port of the burner may be propagated to the exit port of the remaining burners through a flame propagation port between the plurality of burners 141. The propagated flame may burn the fuel that has passed through the remaining burners. In addition, a flame detector 140b may be adjacent to the exit port of the burner located at the other end of the plurality of burners 141. When the flame detector 140b detects a flame, it may be considered that a flame according to the combustion reaction is formed in the remaining burners due to the characteristics of the flame propagation described above.

The heat exchanger 150 may be located in the second space 102S between the top plate 10b and the bottom plate 10c. The heat exchanger 150 may provide a passage for the combustion gas. One end of the heat exchanger 150 may be coupled to the right side of the first plate 111 of the mount plate 110. The other end of the heat exchanger 150 may be spaced apart from the one end of the heat exchanger 150, and may be coupled to the right side of the first plate 111.

In addition, a plurality of heat exchangers 150 may be provided. The number of heat exchangers 150 may be equal to the number of burners 141. Each of the plurality of heat exchangers 150 may be connected to each of the plurality of burners 141. The plurality of heat exchangers 150 may be spaced apart from each other in the front-rear direction.

In addition, the heat exchanger 150 may be a tubular type heat exchanger. The heat exchanger 150 may include a first tube 150a, a bend 150b, and a second tube 150c. The passage of the combustion gas may be formed in the inside of the first tube 150a, the inside of the bend 150b, and the inside of the second tube 150c. For example, the diameter of the first tube 150a may be substantially equal to the diameter of the bend 150b and the diameter of the second tube 150c.

The first tube 150a may extend long in the left-right direction. The left end of the first tube 150a may form the one end of the heat exchanger 150, and may be referred to as an entrance port of the heat exchanger 150. The entrance port of the heat exchanger 150 may communicate with the burner 141 through a first hole (not shown) formed in the first plate 111.

The second tube 150c may extend long in the left-right direction. The second tube 150c may be spaced apart from the first tube 150a toward the upper side. The left end of the second tube 150c may form the other end of the heat exchanger 150, and may be referred to as an exit port of the heat exchanger 150. The exit port of the heat exchanger may communicate with the inside of the collect box 160 described later through a second hole (not shown) formed in the first plate 111.

The bend 150b may be connected to the right end of the first tube 150a and the right end of the second tube 150c. The bend 150b may be convex to the right. The bend 150b may transmit the combustion gas passing through the first tube 150a to the second tube 150c. Accordingly, the combustion gas may flow to the right in the first tube 150a, and may flow to the left in the second tube 150b. The bend 150b may be referred to as a U-shaped bend.

The collect box 160 may be located in the upper side of the burner box 140, and may be installed in the left side of the first plate 111 of the mount plate 110. The combustion gas passing through the heat exchanger 150 may flow into the inside of the collect box 160.

The inducer 170 may be installed in the left side of the collect box 160. The entrance port of the inducer 170 may communicate with the inside of the collect box 160. The exit port 171 of the inducer 170 may be connected to an exhaust pipe 180 (see FIG. 2). The inducer 170 may cause the flow of combustion gas that passes through the heat exchanger 150, the collector box 160, the inducer 170, and the exhaust pipe 180. In addition, the inducer 170 may cause the flow of the fluid that passes through the burner 141. Meanwhile, the inducer 170 may be referred to as a fan.

The exhaust pipe 180 (see FIG. 2) may extend upwardly from the exit port 171 of the inducer 170. The exhaust pipe 180 may penetrate the second plate 112 of the mount plate 110, the horizontal plate 10a, and the first long side LS1, and may discharge the combustion gas to the outside. The combustion gas flowing through the exhaust pipe 180 may be referred to as exhaust gas. For example, the temperature of the exhaust gas may be about 250 ~ 300 °C.

Accordingly, the air discharged from the blower 16 may pass around the heat exchanger 150 via the top hole 100a, and may be supplied into the room through the bottom hole 100b. Here, the bottom hole 100b may be the first outlet described above with reference to FIGS. 1 and 2. At this time, the air passing around the heat exchanger 150 may receive heat energy from the combustion gas flowing along the heat exchanger 150. That is, the temperature of the air may be increased while passing around the heat exchanger 150.

The ventilation device 10 may or may not include the gas furnace 100.

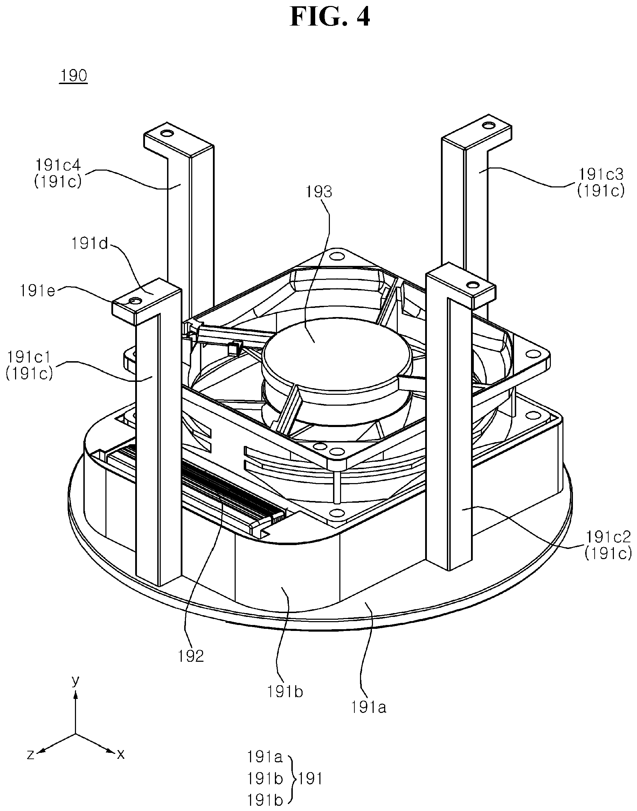

Referring to FIGS. 1 and 4, an ion generating device 190 may be mounted inside the top part 10T which is a portion forming the first long side LS1 of the housing 10H. The ion generating device 190 may be referred to as an ion supply device or a disinfection device.

The ion generating device 190 may include a bracket 191, an ionizer 192, and a fan 193. The bracket 191 may be fixed to the inside of the housing 10H, and the ionizer 192 and the fan 193 may be detachably coupled to the bracket 191.

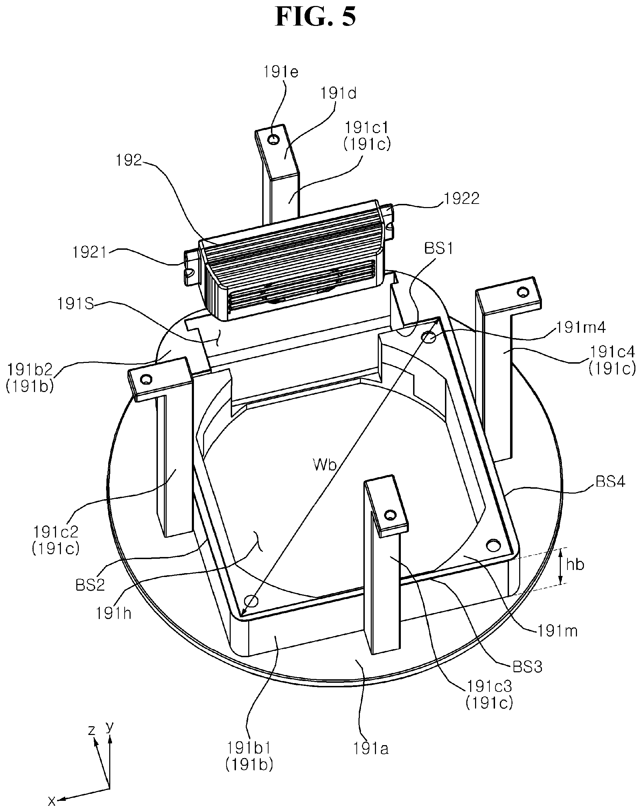

Referring to FIG. 5, the bracket 191 may include a base 191a, a body 191b, and a plurality of legs 191c.

The base 191a may form a lower surface of the bracket 191. The base 191a may have a ring shape as a whole. That is, in the up-down direction, a discharge hole 191h may penetrate the upper and lower surfaces of the bracket 191. The base 191a may be referred to as a ring plate or a bottom plate.

The body 191b may protrude upward from the upper surface of the base 191a. The body 191b may have a hollow block shape as a whole. That is, the body 191b may be opened up and down. In the up-down direction, the discharge hole 191h may penetrate the upper and lower surfaces of the body 191b. The body 191b may be referred to as a block. In addition, the body 191b may include a seating portion 191b1 and a receiving portion 191b2. All parts of the seating portion 191b1 and the receiving portion 191b2 may be located on the base 191a.

The seating portion 191b1 may have four sides BS1, BS2, BS3, BS4, that are orthogonal to each other. The aforementioned discharge hole 191h may be formed in the seating portion 191b1. A diagonal length wb of the seating portion 191b1 may be greater than a height hb of the seating portion 191b1.

The receiving portion 191b2 may protrude from the first side BS1 of the seating portion 191b1 in the radial direction of the base 191a. The receiving portion 191b2 may extend along the first side BS1, and may be formed as one body with the second side BS2 and the fourth side BS4 of the seating portion 191b1. Here, the second side BS2 and the fourth side BS4 may be connected to the first side BS1, and may face each other with respect to the first side BS1. The height of the receiving portion 191b2 may be equal to the height hb of the seating portion 191b1.

The slot 191S may be formed inside the receiving portion 191b2 from the upper surface of the receiving portion 191b2. A portion of the first side BS1 may be cut-out, and the slot 191S may communicate with the discharge hole 191h through the portion of the first side BS1. The shape of the slot 191S may correspond to the shape of the ionizer 192.

In this case, the ionizer 192 may be detachably inserted into the slot 191S. That is, the ionizer 192 may be located between the inner surface and the outer surface of the body 191b. The ionizer 192 inserted into the slot 191S may be detachably coupled to the inside of the receiving portion 191b2 through a coupling portion 1921, 1922. The ionizer 192 coupled to the receiving portion 191b2 may communicate with the discharge hole 191h.

The plurality of legs 191c may be fixed to the upper surface of the base 191a. The plurality of legs 191c may be located around the body 191b. For example, the first leg 191c1 may face the first side BS1 with respect to the receiving portion 191b2. In addition, each of the second leg 191c2, the third leg 191c3, and the fourth leg 191c4 may face each of the second side BS2, the third side BS3, and the fourth side BS4.

In addition, the plurality of legs 191c may extend in the up-down direction. The height of the plurality of legs 191c may be greater than the sum of the height hb of the body 191b and the height of the fan 193 (see FIG. 4).

In addition, a foot 191d may be bent outside the bracket 191 at the upper end of the leg 191c. The foot 191d may be orthogonal to the leg 191c, and may contact the inside of the top part 10T (see FIG. 1) which is a portion forming the first long side LS1 of the housing 10H. A fastening member such as a screw may be coupled to the inside of the housing 10H through a hole 191e formed in the foot 191d.

Accordingly, the bracket 191 may be detachably coupled to the inner side of the housing 10H. In this case, the components (see FIG. 4) of the ion generating device 190 excluding the foot 191d may be spaced apart downward from the inside of the housing 10H.

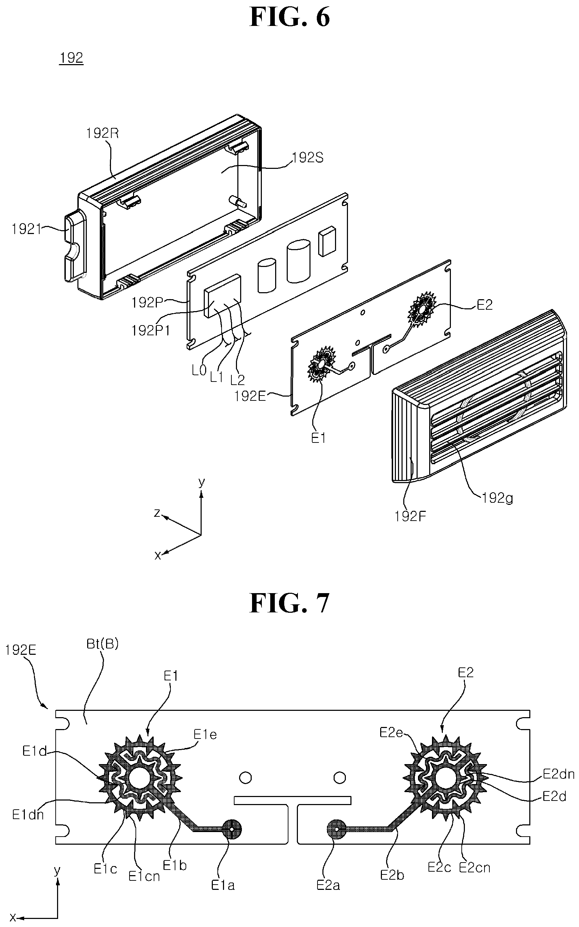

Referring to FIG. 6, the ionizer 192 may include a case 192R, 192F, a voltage generator 192P, and an ion generator 192E.

The case 192R, 192F may be extended long. The case 192R, 192F may include a rear case 192R and a front case 192F that are detachably coupled to each other. An inner space 192S of the case 192R, 192F may be formed between the rear case 192R and the front case 192F. The above-described coupling portion 1921, 1922 (see FIG. 5) may be formed in a side surface of the rear case 192R. A case hole 192g may be formed on the front surface of the front case 192F, and may communicate with the inner space 192S. For example, the front surface of the case 192F may have a grille shape.

The voltage generator 192P may be installed in the inner space 192S, and may be connected to a power source (not shown). The voltage generator 192P may include a printed circuit board PCB (unsigned) and a transformer 192P1 mounted on the PCB. The voltage generator 192P may be electrically connected to an ion generator 192E described later through a wire L1, L2, L0, and may apply a high voltage to the ion generator 192E. The voltage generator 192P may be referred to as a high voltage generator.

The ion generator 192E may be installed in the inner space 192S, and may be located between the voltage generator 192P and the front case 192F. That is, the ion generator 192E may face the case hole 192g. The electrodes E1 and E2 may be formed on the surface of the ion generator 192E. When a high voltage is applied to the electrodes E1 and E2 by the voltage generator 192P, ions may be generated, which will be described in more detail later.

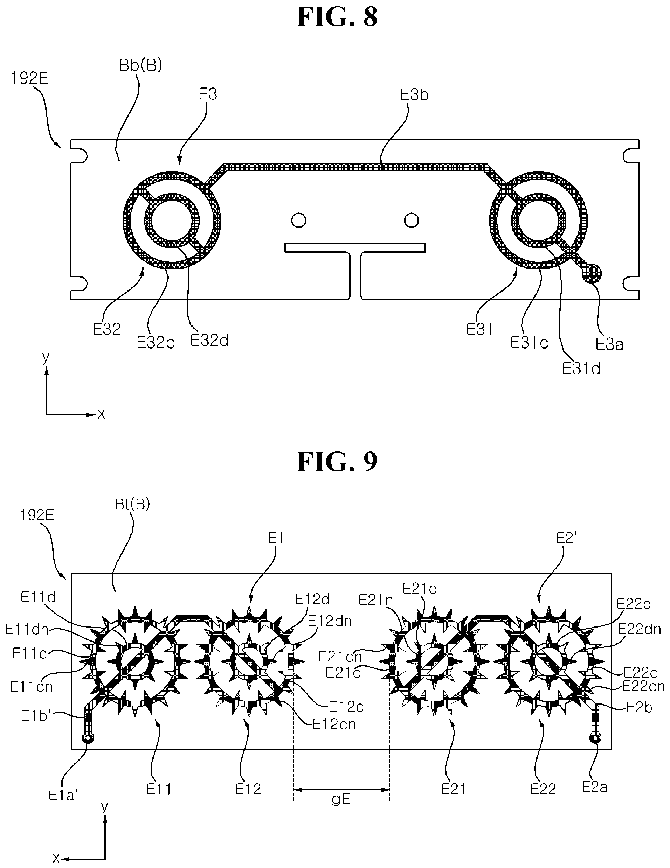

Referring to FIGS. 7 and 8, the ion generator 192E may include a substrate B, a discharge electrode E1, E2, and a ground electrode E3.

The substrate B may be formed of a dielectric substance. For example, the substrate B may include a ceramic or synthetic resin material. The first surface Bt of the substrate B may face the case hole 192g (see FIG. 6), and the second surface Bb of the substrate B may face the voltage generator 192P. The first surface Bt may be referred to as a front surface or an upper surface, and the second surface Bb may be referred to as a rear surface or a lower surface.

The discharge electrode E1, E2 may be formed on the first surface Bt of the substrate B. The discharge electrode E1, E2 may include a metal material such as copper (Cu). For example, the discharge electrode E1, E2 may include a first discharge electrode E1 and a second discharge electrode E2 spaced apart from each other in the length direction of the substrate B. For example, the first discharge electrode E1 and the second discharge electrode E2 may be symmetrical left and right.

The first discharge electrode E1 may include a first point E1a, a first line E1b, a first outer circle E1c, and a first inner circle E1d.

The first point E1a may be connected to a first wire L1 (see FIG. 6), and may be a portion to which the voltage of the voltage generator 192P (see FIG. 6) is applied. The first point E1a may be referred to as a first terminal.

The first line E1b may connect the first point E1a and first circles E1c, E1d.

A first outer circle E1c and a first inner circle E1d may be a concentric circle. A diameter of the first outer circle E1c may be greater than a diameter of the first inner circle E1d. A portion of the aforementioned first line E1b may be connected to the first outer circle E1c and the first inner circle E1d, at between the first outer circle E1c and the first inner circle E1d.

In addition, the first outer circle E1c may include first outer needles E1cn. In addition, the first inner circle E1d may include first inner needles E1dn. For example, the number of the first outer needles E1cn may be greater than the number of the first inner needles E1dn. Meanwhile, the barrier E1e may be located between the first outer circle E1c and the first inner circle E1d, and may minimize discharge interference between the first outer needles E1cn and the first inner needles E1dn.

The second discharge electrode E2 may include a second point E2a, a second line E2b, a second outer circle E2c, and a second inner circle E2d.

The second point E2a may be connected to a second wire L2 (see FIG. 6), and may be a portion to which the voltage of the voltage generator 192P (see FIG. 6) is applied. The second point E2a may be referred to as a second terminal.

The second line E2b may connect the second point E2a and second circles E2c, E2d.

The second outer circle E2c and the second inner circle E2d may be a concentric circle. A diameter of the second outer circle E2c may be greater than a diameter of the second inner circle E2d. A portion of the aforementioned second line E2b may be connected to the second outer circle E2c and the second inner circle E2d, at between the second outer circle E2c and the second inner circle E2d.

In addition, the second outer circle E2c may include second outer needles E2cn. In addition, the second inner circle E2d may include second inner needles E2dn. For example, the number of the second outer needles E2cn may be greater than the number of the second inner needles E2dn. Meanwhile, the barrier E2e may be located between the second outer circle E2c and the second inner circle E2d, and may minimize discharge interference between the second outer needles E2cn and the second inner needles E2dn.

The ground electrode E3 may be formed on the second surface Bb of the substrate B. The ground electrode E3 may include a metal material such as copper (Cu). For example, the ground electrode E3 may include a ground point E3a, a connector E3b, a first ground electrode E31, and a second ground electrode E32. The ground point E3a may be connected to a wire L0 (see FIG. 6). The connector E3b may connect the ground point E3a to the first and second ground electrodes E31 and E32.

In addition, in the thickness direction of the substrate B, the first ground electrode E31 may be aligned with the first discharge electrode E1. The first ground electrode E31 may have a shape corresponding to the first outer circle E1c and the first inner circle E1d of the first discharge electrode E1.

In addition, in the thickness direction of the substrate B, the second ground electrode E32 may be aligned with the second discharge electrode E2. The second ground electrode E32 may have a shape corresponding to the second outer circle E2c and the second inner circle E2d of the second discharge electrode E2.

Accordingly, when a high voltage is applied to the discharge electrodes E1, E2 by the voltage generator 192P, the discharge electrodes E1, E2 may generate negative ions and/or positive ions. That is, the first discharge electrode E1 may be a negative ion discharge electrode generating negative ions, or a positive ion discharge electrode generating positive ions. In addition, the second discharge electrode E2 may be a negative ion discharge electrode generating negative ions, or a positive ion discharge electrode generating positive ions.

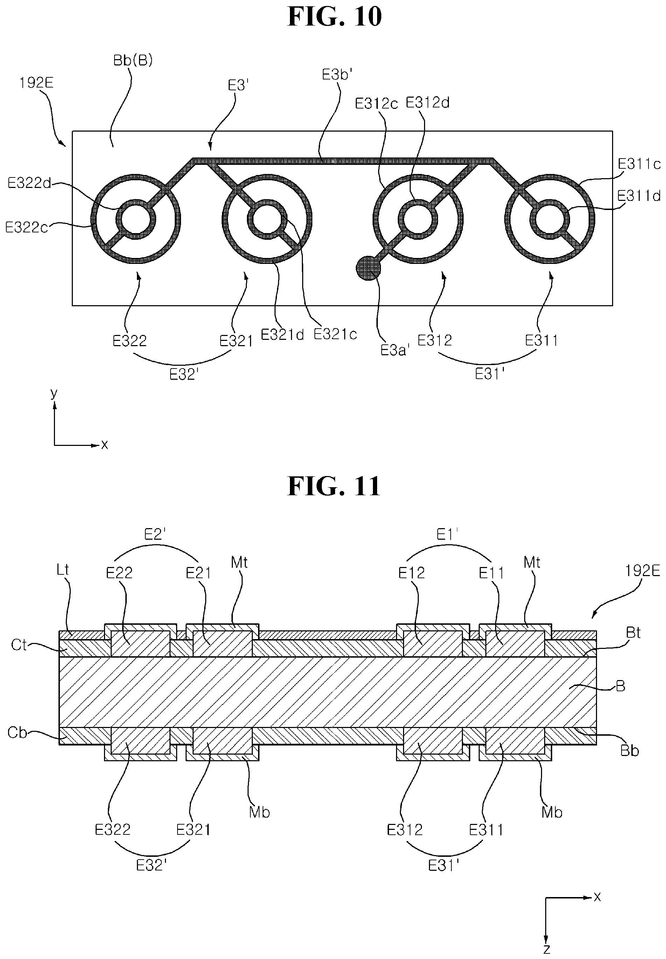

Referring to FIGS. 9 and 10, the ion generator 192E may include a substrate B, a discharge electrode E1', E2', and a ground electrode E3'.

The substrate B may be formed of a dielectric substance. For example, the substrate B may include a ceramic or synthetic resin material. The first surface Bt of the substrate B may face the case hole 192g (see FIG. 6), and the second surface Bb of the substrate B may face the voltage generator 192P. The first surface Bt may be referred to as a front surface or an upper surface, and the second surface Bb may be referred to as a rear surface or a lower surface.

The discharge electrode E1', E2' may be formed on the first surface Bt of the substrate B. The discharge electrode E1', E2' may include a metal material such as copper Cu. For example, the discharge electrode E1', E2' may include a first discharge electrode E1' and a second discharge electrode E2' spaced apart from each other in the length direction of the substrate B (see gE). For example, the first discharge electrode E1' and the second discharge electrode E2' may be symmetrical left and right.

The first discharge electrode E1' may include a first point E1a', a first line E1b', and a pair of first circles E11 and E12. The first point E1a' may be connected to the first wire L1 (see FIG. 6), and may be a portion to which a voltage of the voltage generator 192P (see FIG. 6) is applied. The first point E1a' may be referred to as a first terminal. The first line E1b' may connect the first point E1a' and the pair of first circles E11 and E2.

The pair of first circles E11 and E12 may be spaced apart from each other in the length direction of the substrate B. The pair of first circles E11 and E12 may have shapes corresponding to each other. For example, any one of the pair of first circles E11 and E12 may have a shape which is the shape of the other one that is rotated counterclockwise or clockwise by 90 degrees. In this case, the description of any one of the pair of first circles E11 and E12 may be equally applied to the other one. In addition, the first circle E11, which is one of the pair of first circles E11 and E12, may include a first outer circle E11c and a first inner circle E11d.

The first outer circle E11c and the first inner circle E11d may be concentric. A diameter of the first outer circle E11c may be greater than a diameter of the first inner circle E11d. A portion of the aforementioned first line E1b' may be connected to the first outer circle E11c and the first inner circle E11d at between the first outer circle E11c and the first inner circle E11d.

In addition, the first outer circle E11c may include first outer needles E11cn. In addition, the first inner circle E11d may include first inner needles E11dn. For example, the number of the first outer needles E11cn may be greater than the number of the first inner needles E11dn. Meanwhile, a barrier (not shown) may be located between the first outer circle E11c and the first inner circle E11d, and may minimize discharge interference between the first outer needles E11cn and the first inner needles E11dn.

The second discharge electrode E2' may include a second point E2a', a second line E2b', and a pair of second circles E21 and E22. The second point E2a' may be connected to a second wire L2 (see FIG. 6), and may be a portion to which the voltage of the voltage generator 192P (see FIG. 6) is applied. The second point E2a' may be referred to as a second terminal. The second line E2b' may connect the second point E2a' and the pair of second circles E21 and E22.

The pair of second circles E21 and E22 may be spaced apart from each other in the length direction of the substrate B. The pair of second circles E21 and E22 may have shapes corresponding to each other. For example, any one of the pair of second circles E21 and E22 may have a shape which is the shape of the other that is rotated by 90 degrees counterclockwise or clockwise. In this case, the description of any one of the pair of second circles E21 and E22 may be equally applied to the other one. In addition, the second circle E21, which is any one of the pair of second circles E21 and E22, may include a second outer circle E21c and a second inner circle E21d.

The second outer circle E21c and the second inner circle E21d may be a concentric circle. A diameter of the second outer circle E21c may be greater than a diameter of the second inner circle E21d. A portion of the aforementioned second line E21b may be connected to the second outer circle E21c and the second inner circle E21d at between the second outer circle E21c and the second inner circle E21d.

In addition, the second outer circle E21c may include second outer needles E21cn. In addition, the second inner circle E21d may include second inner needles E21dn. For example, the number of the second outer needles E21cn may be greater than the number of the second inner needles E21dn. Meanwhile, the barrier (unsigned) may be located between the second outer circle E21c and the second inner circle E21d, and may minimize discharge interference between the second outer needles E21cn and the second inner needles E21dn.

The ground electrode E3' may be formed on the second surface Bb of the substrate B. The ground electrode E3' may include a metal material such as copper Cu. For example, the ground electrode E3' may include a ground point E3a', a connector E3b', a first ground electrode E31', and a second ground electrode E32'. The ground point E3a' may be connected to a wire L0 (see FIG. 6). The connector E3b' may connect the ground point E3a' to the first and second ground electrodes E31' and E32'.

In addition, in the thickness direction of the substrate B, the first ground electrode E31' may be aligned with the first discharge electrode E1'. The first ground electrodes E311, E312 may have shapes corresponding to the pair of first circles E11 and E12.

In addition, in the thickness direction of the substrate B, the second ground electrode E32' may be aligned with the second discharge electrode E2'. The second ground electrode E321, E322 may have shapes corresponding to the pair of second circles E21 and E22.

Accordingly, when a high voltage is applied to the discharge electrodes E1' and E2' by the voltage generator 192P, the discharge electrodes E1' and E2' may generate negative ions and/or positive ions. That is, the first discharge electrode E1' may be a negative ion discharge electrode that generates negative ions, or a positive ion discharge electrode that generates positive ions. In addition, the second discharge electrode E2' may be a negative ion discharge electrode that generates negative ions, or a positive ion discharge electrode that generates positive ions.

Referring to FIG. 11, a first protective layer Ct may be formed on the first surface Bt of the substrate B, and may be located around the discharge electrodes E1' and E2' or the discharge electrodes E1 and E2 (see FIG. 7). A second protective layer Cb may be formed on the second surface Bb of the substrate B, and may be located around the ground electrodes E31' and E32' or the ground electrodes E31 and E32 (see FIG. 8).

A first coating layer Mt may be formed on the surfaces of the discharge electrodes E1' and E2' or the discharge electrodes E1, E2 (see FIG. 7). A second coating layer Mb may be formed on the surfaces of the ground electrodes E31' and E32' or the ground electrodes E31 and E32 (see FIG. 8). For example, the first coating layer Mt and the second coating layer Mb may include a metal material such as gold Au.

Meanwhile, a photocatalyst Lt may be coated on the surface of the first protective layer Ct. The photocatalyst Lt may include tungsten oxide, titanium oxide, zinc oxide, or zirconium oxide. The photocatalyst Lt may be activated by light. For example, the photocatalyst Lt may be activated by light in an ultraviolet wavelength band.

Accordingly, as a high voltage is applied to the discharge electrodes E1' and E2' or the discharge electrodes E1 and E2 (see FIG. 7), a plasma discharge may be generated, and an ultraviolet light UV generated by this can activate the photocatalyst Lt. In this case, radicals and ions may be generated, and oxidation of organic matter may be promoted to help disinfection and deodorization.

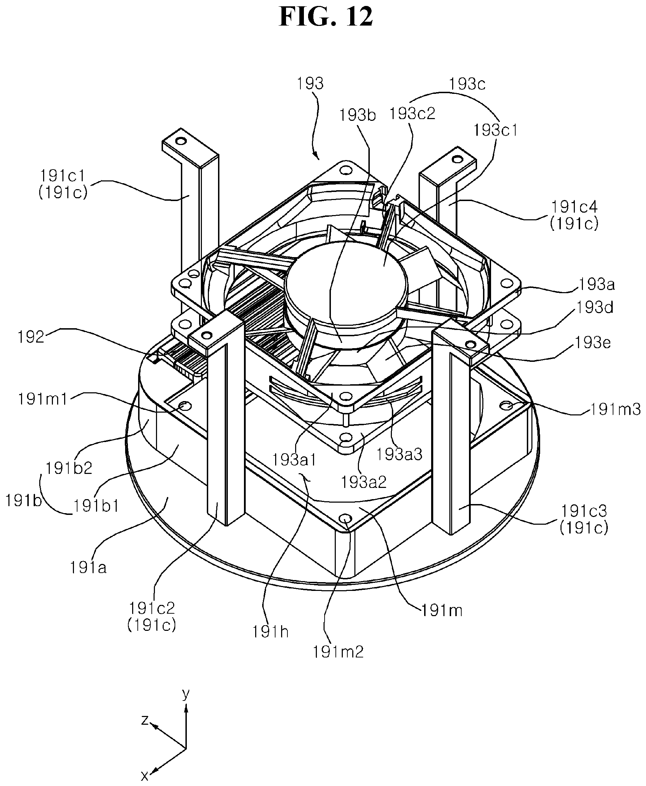

Referring to FIG. 12, the fan 193 may include a fan housing 193a, a motor 193b, a holder 193c, a hub 193d, and a plurality of blades 193e. The fan housing 193a may be opened up and down, and the remaining components of the fan 193 excluding the fan housing 193a may be located in the inner space of the fan housing 193a.

For example, the fan housing 193a may include a first flat portion 193a1, a second flat portion 193a2, and a pillar portion 193a3 formed as one body. The first flat portion 193a1 may form an upper surface of the fan housing 193a, and the second flat portion 193a2 may form a lower surface of the fan housing 193a. The pillar portion 193a3 may be located between the first flat portion 193a1 and the second flat portion 193a2, and may have a flat cylinder shape. The inner space of the fan housing 193a may be formed by penetrating the first flat portion 193a1, the pillar portion 193a3, and the second flat portion 193a2 in the up-down direction. The inner space may communicate with the discharge hole 191h.

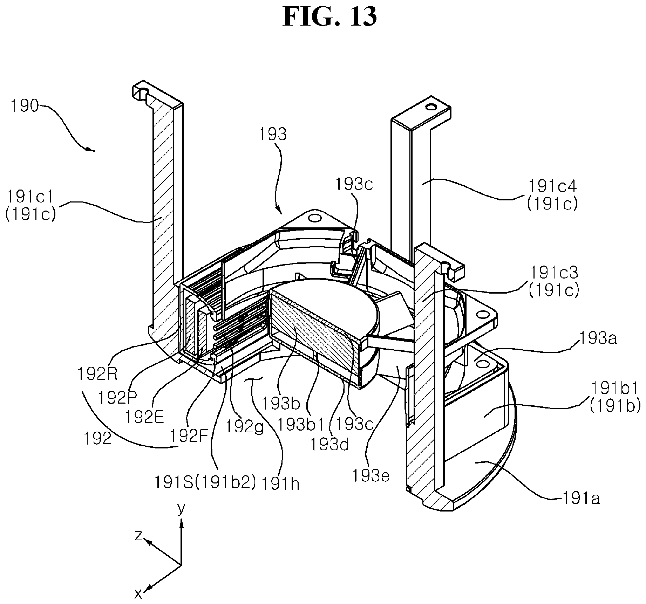

The motor 193b may provide a rotational force. The motor 193b may be an axial fan motor. The motor 193b may be located in the inner space of the fan housing 193a. The rotation shaft 193b1 (see FIG. 13) of the motor 193b may extend downward from the motor 193b. The rotation shaft 193b1 of the motor 193b may be coaxial with the central axis of the fan 193.

One side of the holder 193c may be fixed to the upper surface of the motor 193b, and the other side of the holder 193c may be fixed to the inner side of the fan housing 193a.

For example, the holder 193c may include a cap 193c1 and arms 193c2. The cap 193c1 may cover the upper surface of the motor 193b, and the motor 193b may be fixed thereto. The arms 193c2 may protrude from the side surface of the cap 193c1 to the inner side of the fan housing 193a, and may be fixed to the inner side of the fan housing 193a. These arms 193c2 may be spaced apart from each other in the circumferential direction of the cap 193c1, and may minimize the flow resistance of the air passing around the arms 193c2.

The hub 193d may be located in the lower side of the motor 193b, and may be fixed to the rotation shaft 193b1 (see FIG. 13) of the motor 193b. The hub 193b may have a cup shape as a whole.

The plurality of blades 193e may be formed on the outer circumferential surface of the hub 193d, and may be spaced apart from each other in the circumferential direction of the hub 193d. The distal end of the blade 193e may be spaced apart from the inner side of the fan housing 193a.

Accordingly, when the motor 193b is driven, the plurality of blades 193e may rotate in the rotational direction of the rotation shaft 193b1 (see FIG. 13). At this time, the air located in the upper side of the fan 193 may be flowed in the axial direction of the fan 193, and may be discharged to the lower side of the fan 193.

Referring to FIGS. 12 and 13, a groove 191m may be formed while being recessed downward from the upper surface of the seating portion 191b1, and may extend along the circumference of the seating portion 191b1. The plurality of fastening holes 191m1, 191m2, 191m3, and 191m4 (see FIGS. 5 and 12) may be formed on the groove 191m, and may be adjacent to corners of the groove 191m. In the up-down direction, the groove 191m may be aligned with the lower surface of the second flat portion 193a2.

Accordingly, the second flat portion 193a2 of the fan housing 193a may be seated in the groove 191m. Each of the plurality of fastening members such as a screw or a long bolt may penetrate the first flat portion 193a1 and the second flat portion 193a2, and may be fastened to each of a plurality of fastening holes 191m1, 191m2, 191m3, 191m4.

In this case, in the horizontal direction, the ionizer 192 coupled to the receiving portion 191b2 may be located outside the fan 193 coupled to the body 191b1. In addition, in the vertical direction, the case hole 192g of the ionizer 192 may be located in the lower side of the fan 193.

Accordingly, the ions generated by the ionizer 192 may be carried by the airflow of the fan 193 and flow to the lower side of the discharge hole 191h. That is, the ions generated by the ionizer 192 may be distributed over an entire disinfection target space (particularly, a part far away from or cornered from the ion generating device) by the fan 193.

Referring back to FIG. 1, the ion generating device 190 may include a first ion generating device 190a and a second ion generating device 190b. The first ion generating device 190a may be located between the recovery wheel 13 and the heat exchanger 14, and may be coupled to the inner side of the top part 10T which is a portion forming the first long side LS1 of the housing 10H. The second ion generating device 190b may be located between the heat exchanger 14 and the reheater 15, and may be coupled to the inner side of the top part 10T which is a portion forming the first long side LS1 of the housing 10H.

Meanwhile, in some embodiment, any one of the first ion generating device 190a and the second ion generating device 190b may be omitted. At this time, considering that a space in which the first ion generating device 190a is installed is located upstream of a space in which the second ion generating device 190b is installed, preferably, the first ion generating device 190a may be provided in the ventilation device 10.

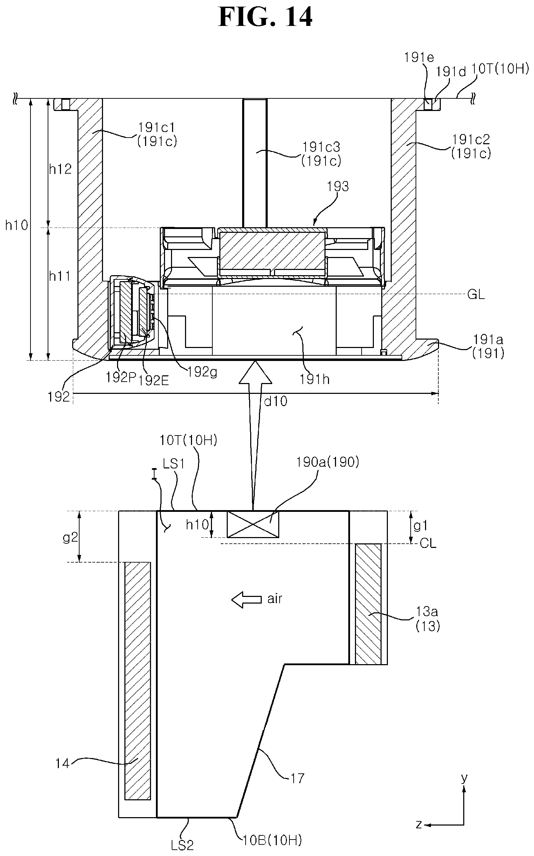

Referring to FIGS. 1 and 14, the first space I may be a portion of the inner space of the housing 10H, and may be a space formed between the first portion 13a of the recovery wheel 13 and the heat exchanger 14. A portion of the top part 10T of the housing 10H, a portion of the bottom part 10B of the housing 10H, and the damper mount 17 may define a portion of the boundary of the first space I.

The upper end of the first portion 13a of the recovery wheel 13 may be spaced downward from the top part 10T. The upper end of the heat exchanger 14 may be spaced downward from the top part 10T. In the up-down direction, a first gap g1 between the top part 10T and the upper end of the first portion 13a ma be smaller than or equal to a second gap g2 between the top part 10T and the upper end of the heat exchanger 14.

The first ion generating device 190a may be coupled to the inner side of the top part 10T between the first portion 13a and the heat exchanger 14. For example, the volume of the first ion generating device 190a may be 0.5% or less of the volume of the first space I. For example, the height h10 of the first ion generating device 190a may be smaller than the first gap g1. That is, the lower end of the first ion generating device 190a may be located in the upper side of the upper end of the first portion 13a and the upper end of the heat exchanger 14. As another example, the height h10 of the first ion generating device 190a may be equal to or slightly larger than the first gap g1. That is, the lower end of the first ion generating device 190a may be located parallel to or slightly lower than the upper end of the first portion 13a.

Accordingly, the first ion generating device 190a may be spaced apart from the main airflow of air sequentially passing through the first portion 13a and the heat exchanger 14 by the blower 16. In other words, in an air conditioning mode, an increase in air flow resistance by the first ion generating device 190a can be minimized. In addition, particularly during a cooling operation, the first space I may be a space having a low temperature and low humidity, and may be a good environment for microorganisms or bacteria to grow. That is, the first ion generating device 190a may remove microorganisms or bacteria inhabiting the first space I by providing ions to the first space I.

Meanwhile, the height h10 of the first ion generating device 190a may be the sum of the first height h11 and the second height h12. The first height h11 may be a distance between the lower end of the base 191a and the upper end of the fan 193. The second height h12 may be a distance between the upper end of the fan 193 and the upper end of the foot 191d. In other words, the upper end of the fan 193 may be spaced downward from the top part 10T by a second height h12.

Accordingly, air may be flowed in the axial direction of the fan 193 through between the top part 10T and the upper end of the fan 193.

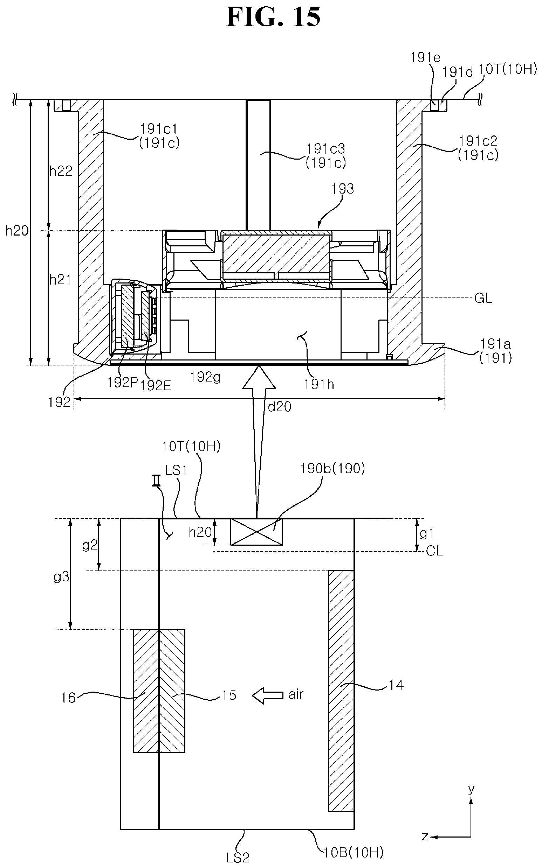

Referring to FIGS. 1 and 15, the second space II may be a portion of the inner space of the housing 10H, and may be a space in which the heat exchanger 14 and the reheater 15 are disposed. A portion of the top part 10T of the housing 10H and a portion of the bottom part 10B of the housing 10H may define a portion of a boundary of the second space II.

The reheater 15 may be spaced downward from the top part 10T. In the up-down direction, a third gap g3 between the top part 10T and the upper end of the reheater 15 may be greater than the second gap g2 between the top part 10T and the upper end of the heat exchanger 14.

The second ion generating device 190b may be coupled to the inner side of the top part 10T between the heat exchanger 14 and the reheater 15. For example, the volume of the second ion generating device 190b may be 0.5% or less of the volume of the second space II. For example, the height h20 of the second ion generating device 190b may be smaller than the second gap g2. That is, the lower end of the second ion generating device 190b may be located in the upper side of the upper end of the reheater 15 and the upper end of the heat exchanger 14. As another example, the height h20 of the second ion generating device 190b may be equal to or slightly larger than the second gap g2. That is, the lower end of the second ion generating device 190b may be located parallel to or slightly lower than the upper end of the heat exchanger 14.

Accordingly, the second ion generating device 190b may be spaced apart from the main airflow of air sequentially passing through the heat exchanger 14 and the reheater 15 by the blower 16. In other words, in the air conditioning mode, an increase in air flow resistance by the second ion generating device 190b can be minimized. In addition, particularly during a cooling operation, the second space II may be a space having a fairly low temperature and a fairly low humidity, and may be a good environment for microorganisms or bacteria to grow. That is, the second ion generating device 190b may remove microorganisms or bacteria inhabiting the second space II by providing ions to the second space II.

Meanwhile, the height h20 of the second ion generating device 190b may be the sum of the first height h21 and the second height h22. The first height h21 may be a distance between the lower end of the base 191a and the upper end of the fan 193. The second height h22 may be a distance between the upper end of the fan 193 and the upper end of the foot 191d. In other words, the upper end of the fan 193 may be spaced downward from the top part 10T by a second height h22.

Accordingly, air may be flowed in the axial direction of the fan 193 through between the top part 10T and the upper end of the fan 193. For example, the height h20 of the second ion generating device 190b may be equal to the height h10 (see FIG. 14) of the first ion generating device 190a.

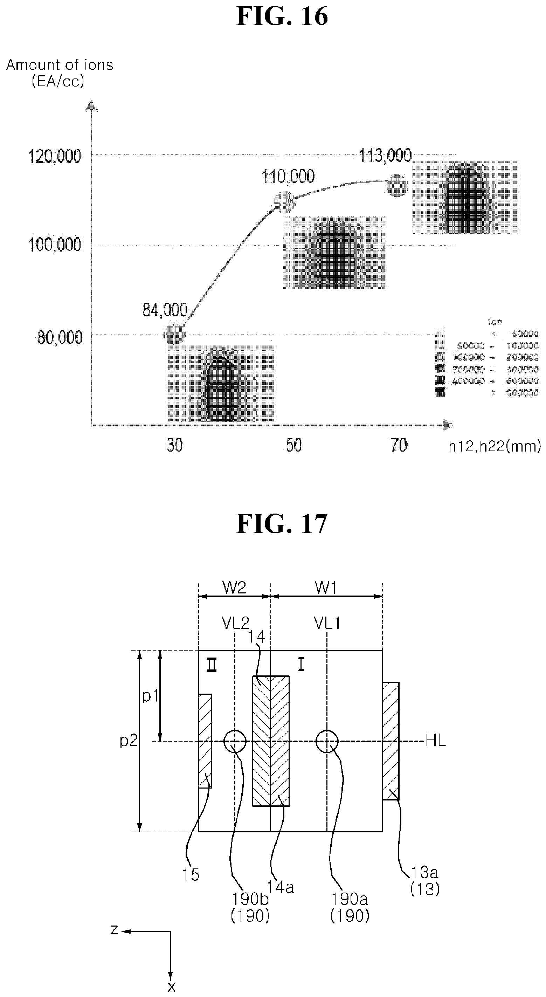

Referring to FIG. 16, it can be seen that the amount of ions (EA/cc) generated by the ion generating device 190a, 190b varies according to the second height h12, h22 described above with reference to FIGS. 14 and 15.

Specifically, when the second height h12, h22 is 30 mm, ions of 84,000 EA/cc may be generated by the ion generating device 190a, 190b. When the second height h12, h22 is 50 mm, ions of 110,000 EA/cc may be generated by the ion generating device 190a, 190b. When the second height h12, h22 is 70 mm, 113,000 EA/cc of ions may be generated by the ion generating device 190a, 190b. That is, as the second height h12, h22 increase, the amount of ions EA/cc generated by the ion generating device 190a, 190b may increase, but may be gradually saturated. For example, the second height h12, h22 may be 50 mm or more.

Referring to FIG. 17, the first space I may be larger than the second space II. In the front-rear direction, the width w1 of the first space I may be greater than the width w2 of the second space II. In the left-right direction, the length p2 of the first space I may be equal to the length p2 of the second space II.

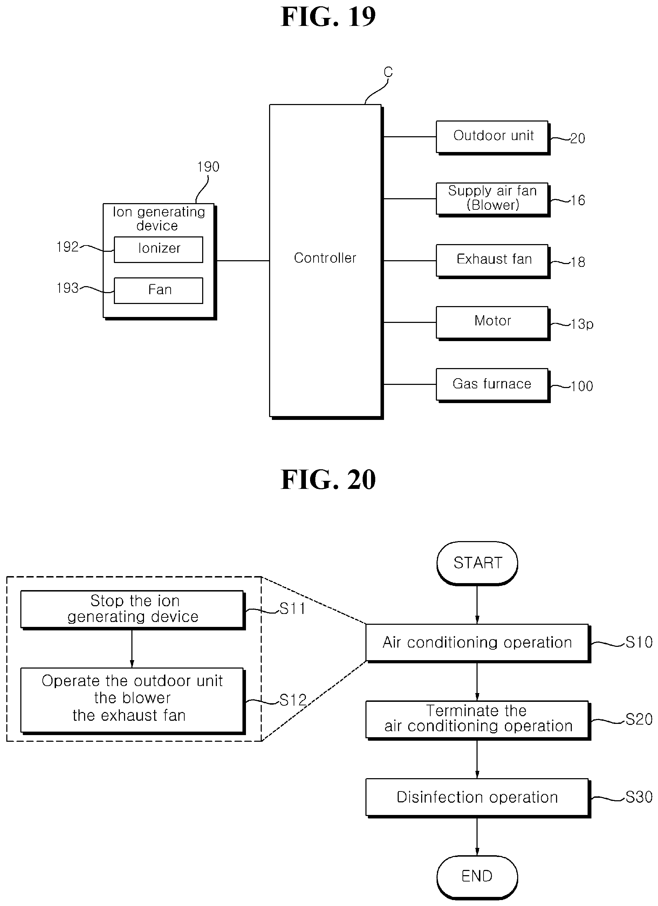

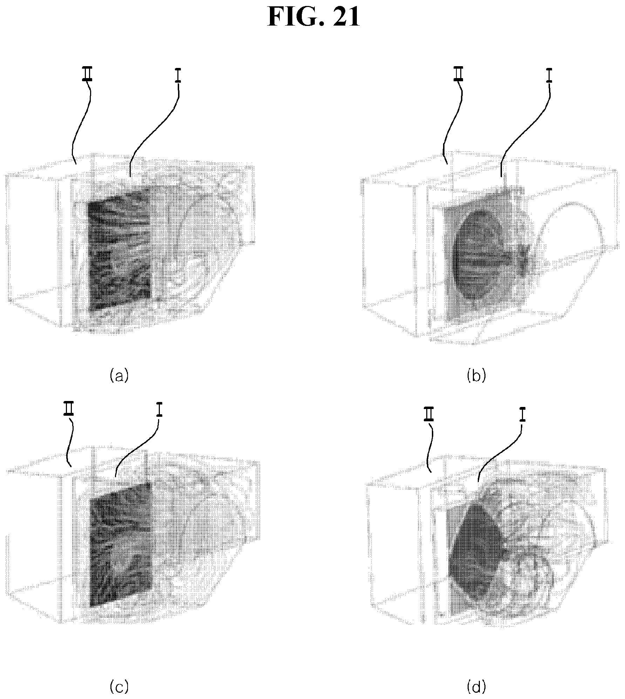

The virtual center line HL may pass through the center (see P1) of the top part 10T (see FIG. 20) defining the upper boundary of the first space I and the center (see P1) of the top part 10T (see FIG. 21) defining the upper boundary of the second space II, and may extend in the front-rear direction.

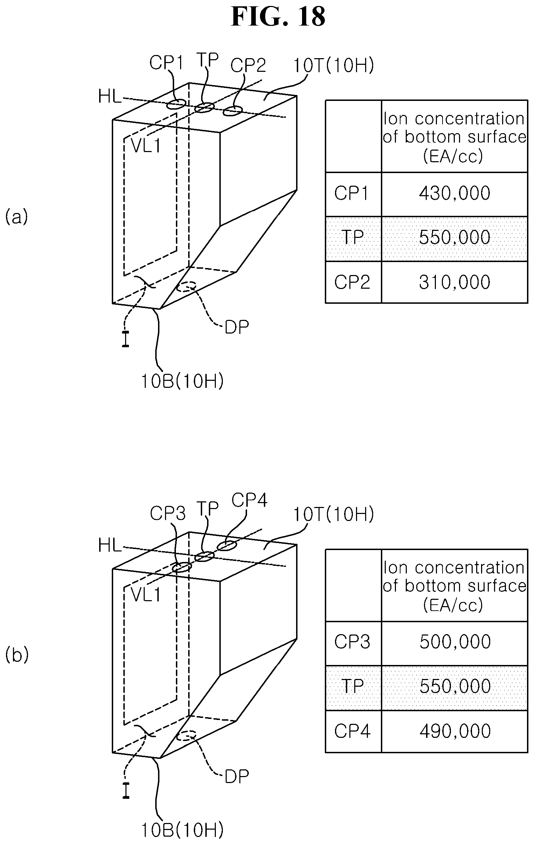

The virtual first line VL1 may pass through the center of the top part 10T (see FIG. 18) defining the upper boundary of the first space I, and may extend in the left-right direction. The virtual second line VL2 may pass through the center of the top part 10T (see FIG. 19) defining the upper boundary of the second space II, and may extend in the left-right direction.

That is, the center line HL and the first line VL1 may be orthogonal at the center of the top part 10T defining the upper boundary of the first space I. Moreover, the center line HL and the second line VL2 may be orthogonal at the center of the top part 10T defining the upper boundary of the second space II.

Referring to FIGS. 17 and 18, it can be seen that the ion concentration EA/cc of the bottom surface varies according to the locations of the first ion generating device 190a and the second ion generating device 190b. For example, the ion concentration EA/cc of the bottom surface of the first space I may be measured at a point DP on the bottom part 10B defining the lower boundary of the first space I.

Referring to FIG. 18A, for example, the ion concentration of the bottom surface according to the location of the first ion generating device 190a in the center line HL may be checked. A target point TP may be located at an intersection of the center line HL and the first line VL1. A first comparison point CP1 and a second comparison point CP2 may be located in the center line HL, and may face each other with respect to the target point TP. When the first ion generating device 190a is disposed in the target point TP, it can be seen that the ion concentration of the bottom surface is measured to be high, in comparison with a case where the first ion generating device 190a is disposed in the first comparison point CP1 or the second comparison point CP2.

Referring to FIG. 18B, for example, the ion concentration of the bottom surface according to the location of the first ion generating device 190a in the first line VL1 may be checked. A target point TP may be located at an intersection of the center line HL and the first line VL1. A third comparison point CP3 and a fourth comparison point CP4 may be located in the first line VL1, and may face each other with respect to the target point TP. When the first ion generating device 190a is disposed in the target point TP, it can be seen that the ion concentration of the bottom surface is measured to be high, in comparison with a case where the first ion generating device 190a is disposed in the third comparison point CP3 or the fourth comparison point CP4.

Accordingly, preferably, the first ion generating device 190a may be disposed in the center of the top part 10T (see FIG. 14) defining the upper boundary of the first space I. Likewise, preferably, the second ion generating device 190b may be disposed in the center of the top part 10T (see FIG. 15) defining the upper boundary of the second space II.

Referring to FIG. 19, the controller C of the air conditioner may be electrically connected to components of the air conditioner.

The controller C may be electrically connected to the outdoor unit 20 and may control the operation of a compressor of the outdoor unit 20. The controller C may be electrically connected to the blower 16 and the exhaust fan 18, and may control the operations of the blower 16 and the exhaust fan 18. The controller C may be electrically connected to the motor 13p, and may control the operation of the recovery wheel 13 through the motor 13p. The controller C may be electrically connected to the gas furnace 100, and may control the operation of the gas furnace 100. Alternatively, the gas furnace 100 may be omitted.

In addition, the controller C may be electrically connected to the ion generating device 190, and may control the operations of the ionizer 192 and the fan 193.

Referring to FIGS. 20 and 21, when the entry condition of the air conditioning mode is satisfied, the controller C may perform an air conditioning operation through the air conditioner 1 (see FIG. 1) (S10). For example, the entry condition of the air conditioning mode may be satisfied according to a user's desire. For another example, the entry condition of the air conditioning mode may be satisfied, when a difference between a desired indoor temperature input to a thermostat in a room and a current indoor temperature detected by a thermocouple of the thermostat exceeds a reference range.

In the air conditioning operation (S10), the controller (C) may stop the operation of the ion generating device 910 or maintain the stopped state (S11), and may operate the outdoor unit 20, the blower 16, and the exhaust fan 18 (S12). Accordingly, the air conditioner 1 may heat and cool, or ventilate the indoor space.

When the termination condition of the air conditioning mode is satisfied, the controller C may terminate the air conditioning operation (S20). For example, the termination condition of the air conditioning mode may be satisfied according to a user's desire. For another example, the termination condition of the air conditioning mode may be satisfied, when a difference between a desired indoor temperature input to a thermostat in a room and a current indoor temperature detected by a thermocouple of the thermostat is within a reference range for a certain period of time.

To terminate the air conditioning operation (S20), the controller C may stop the operation of the outdoor unit 20, the blower 16, and the exhaust fan 18.

When the air conditioning operation is terminated (S20), the controller C may operate the ion generating device 910 to perform the disinfection operation (S30). In the disinfection operation S30, the controller C may operate the ionizer 192 and the fan 193 for a certain period of time (e.g. 90 minutes). Accordingly, the air conditioner 1 may disinfect the inside of the ventilation device 10 (see FIG. 1).

Referring to FIGS. 21A and 21B, for example, the ion generating device 190 may be disposed in the first space I, and may provide ions to the filter 14a (see FIG. 17). The axial direction of the fan 193 of the ion generating device 190 may be orthogonal to the filter 14a. At this time, when not only the fan 193 but also the blower 16 is operated, the area through which ions pass in the total area of the filter 14a is 5.65%, whereas when the fan 193 is operated, but the blower 16 is stopped, the area through which ions pass in the total area of the filter 14a may be 51.18%.

Referring to FIGS. 21C and 21D, for example, the ion generating device 190 may be disposed in the first space I, and provide ions to the heat exchanger 14 (see FIG. 17) (i.e. filter 14a may be omitted). The axial direction of the fan 193 of the ion generating device 190 may be orthogonal to the heat exchanger 14. At this time, when not only the fan 193 but also the blower 16 is operated, the area through which ions pass in the total area of the heat exchanger 14 is 6.05%, whereas when the fan 193 is operated but the blower 16 is stopped, the area through which ions pass in the total area of the heat exchanger 14 may be 58.44%.

Accordingly, in the disinfection operation S30, when the ionizer 192 and the fan 193 of the ion generating device 190 are operated but the blower 16 is stopped, the disinfection performance may be improved.

In addition, the contents described above with reference to FIG. 21 may be identically applied to the disinfection operation (S30) of the ion generating device 190a, 190b coupled to the inner side of the top part 10T (see FIGS. 14 and 15). That is, in the disinfection operation S30, in order to improve the disinfection performance and evenly distribute the ions in a disinfection space, the ionizer 192 and the fan 193 of the ion generating device 190a, 190b coupled to the inner side of the top part 10T may be operated but the blower 16 may be stopped.

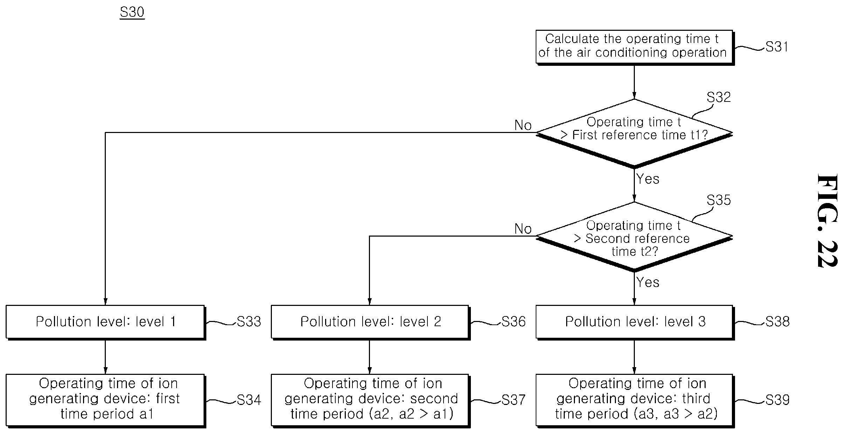

Referring to FIG. 22, in the disinfection operation S30, the controller C may calculate the operating time t of the air conditioning operation performed before the disinfection operation (S31). The operating time t may be a time interval between a start time and a termination time of the air conditioning operation, and may be measured by a timer (not shown) electrically connected to the controller C.

The controller C may determine whether the operating time t exceeds a first reference time t1 (S32). The first reference time t1 may be information input and/or stored in the controller C. For example, the first reference time t1 may be adjusted by a user. For example, the first reference time t1 may be 7 hours.

If it is determined at S32 that the operating time t is less than or equal to the first reference time t1 (No at S32), the controller C may determine that the pollution level of the air conditioner (particularly, the ventilation device) is level 1 (S33). The pollution level may indicate the degree of pollution by bacteria or microorganisms accumulated in the ventilation device in response to the air conditioning operation, and it may be determined (estimated) that the pollution degree becomes severe as the pollution level becomes higher.

After or simultaneously with S33, the controller C may operate the ion generating device 190 for a first time period a1 (S34). In the disinfection operation (S30), the operating time of the ion generating device 190 may increase as the pollution level increases. For example, the first time period a1 may be 90 minutes.

When it is determined at S32 that the operating time t exceeds the first reference time t1 (Yes at S32), the controller C may determine whether the operating time t exceeds a second reference time t2 (S35). The second reference time t2 may be information input and/or stored in the controller C. For example, the second reference time t2 may be adjusted by a user. For example, the second reference time t2 may be 14 hours.

When it is determined at S35 that the operating time t is less than or equal to the second reference time t2 but exceeds the first reference time t1 (No at S35), the controller C may determine that the pollution level of the air conditioner (particularly, the ventilation device) is level 2 (S36). Pollution level 2 may be more sever in pollution degree than pollution level 1.

After or simultaneously with S36, the controller C may operate the ion generating device 190 for a second time period a2 (S37). The second time period a2 may be longer than the first time period a1. For example, the second time period a2 may be 180 minutes.

When it is determined at S35 that the operating time t exceeds the second reference time t2 (Yes at S35), the controller C determines that the pollution level of the air conditioner (particularly, the ventilation device) is level 3 (S38). Pollution level 3 may be more severe in pollution degree than pollution level 2.

After or simultaneously with S38, the controller C may operate the ion generating device 190 for a third time period a3 (S39). The third time period a3 may be longer than the second time period a2. For example, the third time period a3 may be 270 minutes.

Accordingly, in the disinfection operation S30, the controller C may adjust the operating time of the ion generating device 190, based on the operating time t information of the air conditioning operation performed before the disinfection operation. That is, since the operating time of the ion generating device 190 is adjusted in stages in response to the operating time t, the disinfection operation can be effectively and efficiently performed.

Referring to FIG. 23, a sensor 199 may be located inside the housing 10H (see FIGS. 1 and 2) adjacent to the first inlet 10i (see FIGS. 1 and 2). The sensor 199 may be located between the first inlet 10i and the radiator 12, and may be located in the supply air passage OA-SA. The sensor 199 may detect (fine) dust in the outdoor air OA flowed in through the first inlet 10i. Alternatively, the sensor 199 may be located in the first space I (see FIG. 17) and/or the second space II (see FIG. 17), etc., and may detect (fine) dust in the air passing through the space in which the sensor 199 is disposed.

The sensor 199 may detect the concentration and/or the number of (fine) dust in the air. The sensor 199 may detect (fine) dust in the air passing through an exit port from an entrance port of the sensor 199 by using a light emitting unit (e.g. IR LED) and a light receiving unit (e.g. photo diode). The sensor 199 may be referred to as a dust sensor or a fine dust sensor.

The controller C of the air conditioner may be electrically connected to the sensor 199, and may obtain information related to the concentration of (fine) dust in the air from the sensor 199.

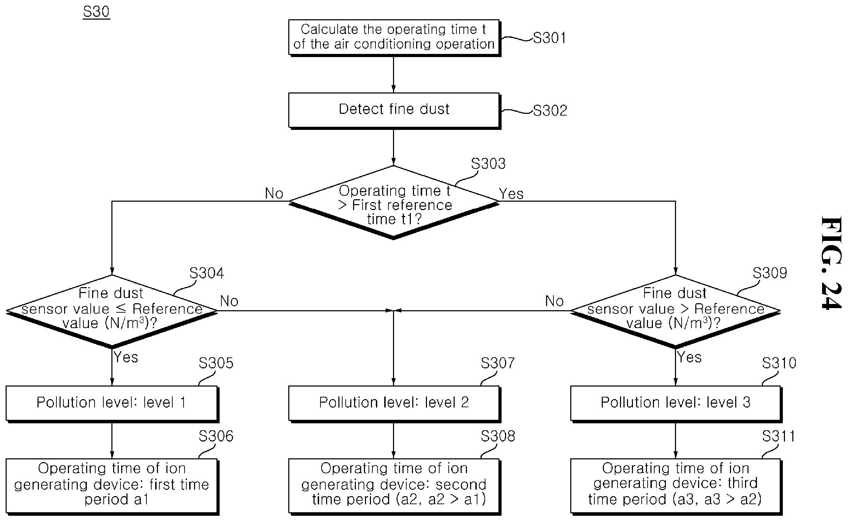

Referring to FIG. 24, in the disinfection operation S30, the controller C may calculate the operating time t of the air conditioning operation performed before the disinfection operation (S301). The operating time t may be a time interval between a start time and a termination time of the air conditioning operation, and may be measured by a timer (not shown) electrically connected to the controller C.

After, before, or simultaneously with S301, the controller C may calculate a sensor value of the fine dust in the outdoor air flowed into the ventilation device based on the information obtained from the sensor 199 (see FIG. 23) (S302). Through the fine dust sensor value, it is possible to determine (estimate) the degree of pollution by bacteria or microorganisms accumulated in the ventilation system in response to the air conditioning operation. For example, bacteria or microorganisms may occupy about 0.1% of fine dust.

For example, the fine dust sensor value may be a value calculated based on a specific time of the air conditioning operation. The fine dust sensor value may be a value calculated based on the start time of the air conditioning operation, the termination time (or the start time of the disinfection operation), or a time point between the start time and the termination time.

As another example, the fine dust sensor value may be an average value of fine dust sensor values detected in real time or at specific time intervals during the air conditioning operation.

The controller C may determine whether the operating time t exceeds the first reference time t1 (S303). The first reference time t1 may be information input and/or stored in the controller C. For example, the first reference time t1 may be adjusted by a user. For example, the first reference time t1 may be 7 hours.

When it is determined at S303 that the operating time t is less than or equal to the first reference time t1 (No at S303), the controller C may determine whether the fine dust sensor value is less than or equal to the reference value (N/m³) (S304). The reference value may be information input and/or stored in the controller C. For example, the reference value may be adjusted by a user. For example, the reference value may be 10⁶/m³ to 10⁸/m³.

When it is determined at S304 that the fine dust sensor value is less than or equal to the reference value (Yes at S304), the controller C may determine that the pollution level of the air conditioner (particularly, the ventilation device) is the first level (S305). The pollution level may indicate the degree of pollution by bacteria or microorganisms accumulated in the ventilation device in response to the air conditioning operation, and it may be determined (estimated) that the pollution degree becomes severe as the pollution level becomes higher.

After or simultaneously with S305, the controller C may operate the ion generating device 190 for the first time period a1 (S306). In the disinfection operation (S30), the operating time of the ion generating device 190 may increase as the pollution level increases. For example, the first time period a1 may be 90 minutes.

When it is determined at S304 that the fine dust sensor value exceeds the reference value (No at S304), the controller C may determine that the pollution level of the air conditioner (particularly, the ventilation device) is level 2 (S307). Pollution level 2 may be more severe in pollution degree than pollution level 1.

After or simultaneously with S307, the controller C may operate the ion generating device 190 for the second time period a2 (S308). The second time period a2 may be longer than the first time period a1. For example, the second time period a2 may be 180 minutes.

When it is determined at S303 that the operating time t exceeds the first reference time t1 (Yes at S303), the controller C may determine whether the fine dust sensor value exceeds the reference value (N/m³) (S309). The reference value may be information input and/or stored in the controller C. For example, the reference value may be adjusted by a user. For example, the reference value may be 10⁶/m³ to 10⁸/m³.

When it is determined at S309 that the fine dust sensor value is less than or equal to the reference value (No at S309), the controller C may perform the aforementioned S307 and S308.

When it is determined at S309 that the fine dust sensor value exceeds the reference value (Yes at S309), the controller C may determine that the pollution level of the air conditioner (particularly, the ventilation device) is level 3 (S310). Pollution level 3 may be more severe in pollution degree than pollution level 2.

After or simultaneously with S310, the controller C may operate the ion generating device 190 for the third time period a3 (S311). The third time period a3 may be longer than the second time period a2. For example, the third time period a2 may be 270 minutes.

Accordingly, in the disinfection operation (S30), the controller C may adjust the operating time of the ion generating device 190, based on the operating time t information of the air conditioning operation performed before the disinfection operation and the fine dust detection information (concentration information). That is, the operating time of the ion generating device 190 is adjusted in stages in response to the operating time t and the concentration of fine dust, thereby effectively and efficiently performing the disinfection operation.

Meanwhile, in some embodiment, the controller C may adjust the operating time of the ion generating device 190, based on the fine dust detection information (concentration information).

Alternatively, the control method described above with reference to FIG. 22 and the control method described above with reference to FIG. 24 may be combined. In the combined control method, if the pollution level by any one of the control method of FIG. 22 and the control method of FIG. 24 is different from the pollution level by the other, a relatively high pollution level may be determined. For example, even if the operating time t is less than or equal to the first reference time t1 (see No at S32 in FIG. 22), when the fine dust sensor value exceeds the reference value (see No at S304 in FIG. 24), the ion generating device may be operated for the second time period a2 in the pollution level 2 (see S307, S308 in FIG. 24). For example, even if the operating time t exceeds the first reference time t1 but is less than or equal to the second reference time t2 (see No at S35 in FIG. 22), when the fine dust sensor value exceeds the reference value (see Yes at S309 in FIG. 24), the ion generating device may be operated for the third time period a3 in the pollution level 3 (see S310, S311 in FIG. 24). For example, even if the fine dust sensor value is less than or equal to the reference value (see No at S309 in FIG. 24), when the operating time t exceeds the second reference time t2 (see Yes at S35 in FIG. 22), the ion generating device may be operated for the third time period a3 in the pollution level 3 (see S38, S39 in FIG. 22).

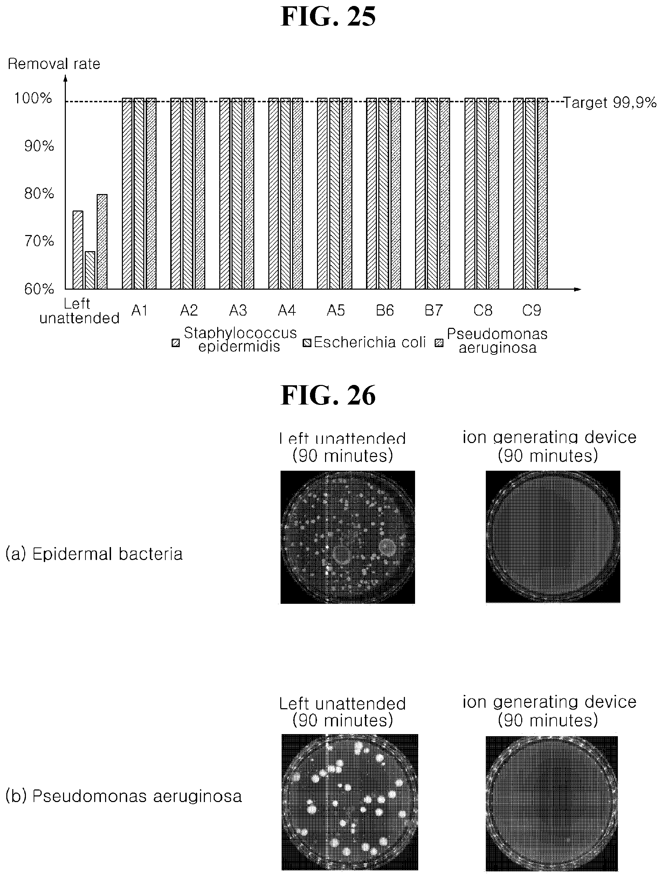

Referring to FIGS. 25 and 26, after the air conditioning operation is completed, the ion generating device 190 is operated to compare the rates of removal of microorganism of a case where a disinfection operation is performed with respect to the air conditioner (particularly, the ventilation device) and a case where the air conditioner (particularly, the ventilation device) is left unattended. In this case, the ion generating device 190 may be disposed in the target point TP, may be coupled to the top part 10T (see FIGS. 17 and 18), and the removal rate of microorganism may be checked in nine points A1, A2, A3, A4, A5, B6, B7, B8, B9 on the bottom part 10B.

Specifically, when the air conditioner (particularly, the ventilation device) is left unattended for 90 minutes without operating the ion generating device 190, it can be seen that Staphylococcus epidermidis is reduced by 76.4%, Escherichia coli is reduced by 67.8%, and Pseudomonas aeruginosa is reduced by 79.8%, in comparison with the time of the termination of the air conditioning operation.

Meanwhile, when the ion generating device 190 is operated for 90 minutes, it can be seen that staphylococcus epidermidis, Escherichia coli, and Pseudomonas aeruginosa are all reduced by 99.9% or more in comparison with the time of the termination of the air conditioning operation.

Accordingly, the ion generating device 190 can effectively remove bacteria or microorganisms remaining in the air conditioner (particularly, the ventilation device) after the air conditioning operation is completed.

The effects of the ion generating device and the air conditioner having the same according to the present invention will be described as follows.

According to at least one of the embodiments of the present invention, it is possible to provide an air conditioner capable of heating or cooling outdoor air through a heat exchanger and supplying it to a room.

According to at least one of the embodiments of the present invention, it is possible to provide an ion generating device capable of removing bacteria or microorganisms propagating in a housing of an air conditioner in which a heat exchanger is installed.

According to at least one of the embodiments of the present invention, it is possible to provide an ion generating device that can be continuously operated for a long time by applying a high voltage to a discharge electrode, and enables each component to be detachably assembled, thereby easily achieving maintenance, management, and repair.

According to at least one of the embodiments of the present invention, a fan of the ion generating device may provide ions generated by the ion generating device to an entire disinfection target space.

According to at least one of the embodiments of the present invention, it is possible to provide an ion generating device having a fan operated independently of a blower for air conditioning operation.

According to at least one of the embodiments of the present invention, since the disinfection operating time is adjusted based on the air conditioning operating time and/or information on fine dust in the air, the disinfection operation can be effectively and efficiently performed.

According to at least one of the embodiments of the present invention, it is possible to provide a coupling structure and an optimal installation location of a ventilation device and an ion generating device of the air conditioner that can maximize the amount of ions generated by the ion generating device.

According to at least one of the embodiments of the present invention, since the ion generating device is located outside of the airflow passing through the heat exchanger, it is possible to minimize air flow resistance during air conditioning operation.

## Claims

1. An air conditioner comprising:
a housing (10H);
a blower (16) configured to cause a flow of air passing through an inner space of the housing;
a heat exchanger (14) located in the inner space of the housing (10H);
an ion generating device (190) spaced apart from the heat exchanger (14) and coupled to an inner side of the housing (10H); and
a controller (C) electrically connected to the blower (16) and the ion generating device (190), and configured to operate the blower (16) in an air conditioning operation and to operate the ion generating device (190) in a disinfection operation;
wherein the ion generating device (190) comprises:
an ionizer (192);
a body (191b) having an inner space (192S) toward which the ionizer (192) faces, as a hollow body; and
a fan (18) coupled to the body (191b) and configured to cause a flow of air passing through the inner space (192S) to cause a flow of an ion generated in the ionizer (192),
wherein one of the blower (16) and the ion generating device (190) is configured to be operated while the other is stopped; and
wherein, in the disinfection operation, the controller (C) is configured to adjust an operating time of the ion generating device (190), based on information on running time of the air conditioning operation performed before the disinfection operation.

2. The air conditioner of claim 1, wherein the controller (C) is configured to perform the disinfection operation after the air conditioning operation is completed.

3. The air conditioner of claim 1 or 2, wherein, in the disinfection operation, the operating time of the ion generating device (190) becomes longer, as the running time becomes longer.

4. The air conditioner of any one of the preceding claims, wherein the controller (C) is configured to operate the ion generating device (190) for a first time when the running time is less than or equal to a first reference time, to operate the ion generating device (190) for a second time when the running time exceeds the first reference time but is less than or equal to a second reference time, and to operate the ion generating device (190) for a third time when the running time exceeds the second reference time,
wherein the second time is greater than the first time, but smaller than the third time.

5. The air conditioner of any one of the preceding claims, further comprising a sensor (199) located in the inner space of the housing (10H) for detecting dust in an air passing through the housing (10H),
wherein the controller (C) is configured to adjust an operating time of the ion generating device (190), based on information obtained from the sensor (199), in the disinfection operation.

6. The air conditioner of claim 5, wherein the operating time of the ion generating device (190) in the disinfection operation becomes longer as a fine dust sensor value which is an amount of dust in the air passing through the housing (10H) becomes larger.

7. The air conditioner of claim 5 or 6, wherein, in the disinfection operation, the controller (C) is configured to adjust an operating time of the ion generating device (190), further based on information on running time of the air conditioning operation performed before the disinfection operation.

8. The air conditioner of claim 6 or 7, wherein the controller (C) is configured to calculate a fine dust sensor value that is an amount of dust in the air passing through the housing (10H), based on the information obtained from the sensor (199),
to operate the ion generating device (190) for a first time, when the running time is less than or equal to a first reference time and the fine dust sensor value is less than or equal to a reference value, and
to operate the ion generating device (190) for a second time, when the running time is less than or equal to the first reference time and the fine dust sensor value exceeds the reference value,
wherein the second time is greater than the first time.

9. The air conditioner of claim 8, wherein the controller (C) is configured to operate the ion generating device (190) for the second time, when the running time exceeds the first reference time and the fine dust sensor value is less than or equal to the reference value, and
to operate the ion generating device (190) for the third time, when the running time exceeds the first reference time and the fine dust sensor value exceeds the reference value,
wherein the third time is greater than the second time.

10. The air conditioner of any one of the preceding claims, wherein the heat exchanger (14) further comprises:
a first heat exchanger (13); and
a second heat exchanger (14) located downstream of the first heat exchanger (13), in a passage of air formed by the fan (18),
wherein the ion generating device (190) is located in a center between the first heat exchanger (13) and the second heat exchanger (14).

11. The air conditioner of claim 10, wherein the heat exchanger (150) further comprises a third heat exchanger (15) located downstream of the second heat exchanger (14), in the passage of air formed by the fan (18),
wherein the ion generating device (190) comprises:
a first ion generating device (190a) located between the first heat exchanger (13) and the second heat exchanger (14); and
a second ion generating device (190b) located between the second heat exchanger (14) and the third heat exchanger (15).

12. The air conditioner of any one of the preceding claims, wherein the housing (10H) comprises a top part (10T) which forms an upper side of the housing (10H), and to which the ion generating device (190) is coupled,
wherein a lower end of the ion generating device (190) is located above an upper end of the heat exchanger (13).

## Patentansprüche

1. Klimaanlage, die Folgendes umfasst:
ein Gehäuse (10H);
ein Gebläse (16), das konfiguriert ist zu bewirken, dass ein Luftstrom durch einen Innenraum des Gehäuses strömt;
einen Wärmetauscher (14), der im Innenraum des Gehäuses (10H) angeordnet ist;
eine Ionenerzeugungsvorrichtung (190), die vom Wärmetauscher (14) beabstandet ist und mit einer Innenseite des Gehäuses (10H) gekoppelt ist; und
eine Steuereinheit (C), die mit dem Gebläse (16) und der Ionenerzeugungsvorrichtung (190) elektrisch verbunden ist und konfiguriert ist, das Gebläse (16) in einem Klimatisierungsbetrieb zu betreiben und die Ionenerzeugungsvorrichtung (190) in einem Desinfektionsbetrieb zu betreiben;
wobei die Ionenerzeugungsvorrichtung (190) Folgendes umfasst:
einen Ionisator (192);
einen Körper (191b) mit einem Innenraum (192S), dem der Ionisator (192) zugewandt ist, als einen Hohlkörper; und
einen Lüfter (18), der mit dem Körper (191b) gekoppelt ist und konfiguriert ist zu bewirken, dass ein Luftstrom durch den Innenraum (192S) strömt, um eine Strömung von Ionen, die im Ionisator (192) erzeugt werden, zu bewirken,
wobei eines bzw. eine des Gebläses (16) und der Ionenerzeugungsvorrichtung (190) konfiguriert ist, betrieben zu werden, während das bzw. die andere angehalten ist; und
wobei die Steuereinheit (C) im Desinfektionsbetrieb konfiguriert ist, eine Betriebszeit der Ionenerzeugungsvorrichtung (190) auf der Grundlage von Informationen über eine Laufzeit des Klimatisierungsbetriebs, der vor dem Desinfektionsbetrieb durchgeführt worden ist, einzustellen.

2. Klimaanlage nach Anspruch 1, wobei die Steuereinheit (C) konfiguriert ist, den Desinfektionsbetrieb durchzuführen, nachdem der Klimatisierungsbetrieb abgeschlossen worden ist.

3. Klimaanlage nach Anspruch 1 oder 2, wobei im Desinfektionsbetrieb die Betriebszeit der Ionenerzeugungsvorrichtung (190) umso länger wird, je länger die Laufzeit wird.

4. Klimaanlage nach einem der vorhergehenden Ansprüche, wobei die Steuereinheit (C) konfiguriert ist, die Ionenerzeugungsvorrichtung (190) für eine erste Zeit zu betreiben, wenn die Laufzeit kleiner oder gleich einer ersten Referenzzeit ist, die Ionenerzeugungsvorrichtung (190) für eine zweite Zeit zu betreiben, wenn die Laufzeit die erste Referenzzeit überschreitet, jedoch kleiner oder gleich einer zweiten Referenzzeit ist, und die Ionenerzeugungsvorrichtung (190) für eine dritte Zeit zu betreiben, wenn die Laufzeit die zweite Referenzzeit überschreitet,
wobei die zweite Zeit länger als die erste Zeit, jedoch kürzer als die dritte Zeit ist.

5. Klimaanlage nach einem der vorhergehenden Ansprüche, die ferner einen Sensor (199) umfasst, der zum Detektieren von Staub in Luft, die durch das Gehäuse (10H) strömt, im Innenraum des Gehäuses (10H) angeordnet ist,
wobei die Steuereinheit (C) konfiguriert ist, im Desinfektionsbetrieb eine Betriebszeit der Ionenerzeugungsvorrichtung (190) auf der Grundlage von Informationen einzustellen, die vom Sensor (199) erhalten werden.

6. Klimaanlage nach Anspruch 5, wobei die Betriebszeit der Ionenerzeugungsvorrichtung (190) im Desinfektionsbetrieb umso länger wird, je größer ein Feinstaubsensorwert ist, der eine Staubmenge in der durch das Gehäuse (10H) strömenden Luft ist.

7. Klimaanlage nach Anspruch 5 oder 6, wobei die Steuereinheit (C) im Desinfektionsbetrieb konfiguriert ist, eine Betriebszeit der Ionenerzeugungsvorrichtung (190) ferner auf der Grundlage von Informationen über eine Laufzeit des Klimatisierungsbetriebs, der vor dem Desinfektionsbetrieb durchgeführt worden ist, einzustellen.

8. Klimaanlage nach Anspruch 6 oder 7, wobei die Steuereinheit (C) konfiguriert ist, auf der Grundlage der Informationen, die vom Sensor (199) erhalten werden, einen Feinstaubsensorwert zu berechnen, der eine Staubmenge in der durch das Gehäuse (10H) strömenden Luft ist,
die Ionenerzeugungsvorrichtung (190) für eine erste Zeit zu betreiben, wenn die Laufzeit kleiner oder gleich einer ersten Referenzzeit ist und der Feinstaubsensorwert kleiner oder gleich einem Referenzwert ist, und
die Ionenerzeugungsvorrichtung (190) für eine zweite Zeit zu betreiben, wenn die Laufzeit kleiner oder gleich der ersten Referenzzeit ist und der Feinstaubsensorwert den Referenzwert überschreitet,
wobei die zweite Zeit länger als die erste Zeit ist.

9. Klimaanlage nach Anspruch 8, wobei die Steuereinheit (C) konfiguriert ist, die Ionenerzeugungsvorrichtung (190) für die zweite Zeit zu betreiben, wenn die Laufzeit die erste Referenzzeit überschreitet und der Feinstaubsensorwert kleiner oder gleich dem Referenzwert ist, und
die Ionenerzeugungsvorrichtung (190) für die dritte Zeit zu betreiben, wenn die Laufzeit die erste Referenzzeit überschreitet und der Feinstaubsensorwert den Referenzwert überschreitet,
wobei die dritte Zeit länger als die zweite Zeit ist.

10. Klimaanlage nach einem der vorhergehenden Ansprüche, wobei der Wärmetauscher (14) ferner Folgendes umfasst:
einen ersten Wärmetauscher (13); und
einen zweiten Wärmetauscher (14), der dem ersten Wärmetauscher (13) nachgelagert in einem Luftkanal, der durch den Lüfter (18) gebildet wird, angeordnet ist,
wobei die Ionenerzeugungsvorrichtung (190) in der Mitte zwischen dem ersten Wärmetauscher (13) und dem zweiten Wärmetauscher (14) angeordnet ist.

11. Klimaanlage nach Anspruch 10, wobei der Wärmetauscher (150) ferner einen dritten Wärmetauscher (15) umfasst, der dem zweiten Wärmetauscher (14) nachgelagert in dem Luftkanal, der durch den Lüfter (18) gebildet wird, angeordnet ist,
wobei die Ionenerzeugungsvorrichtung (190) Folgendes umfasst:
eine erste Ionenerzeugungsvorrichtung (190a), die zwischen dem ersten Wärmetauscher (13) und dem zweiten Wärmetauscher (14) angeordnet ist; und
eine zweite Ionenerzeugungsvorrichtung (190b), die zwischen dem zweiten Wärmetauscher (14) und dem dritten Wärmetauscher (15) angeordnet ist.

12. Klimaanlage nach einem der vorhergehenden Ansprüche, wobei das Gehäuse (10H) einen oberen Abschnitt (10T) umfasst, der eine Oberseite des Gehäuses (10H) bildet und mit dem die Ionenerzeugungsvorrichtung (190) gekoppelt ist,
wobei ein unteres Ende der Ionenerzeugungsvorrichtung (190) über einem oberen Ende des Wärmetauschers (13) angeordnet ist.

## Revendications

1. Climatiseur comportant :
un caisson (10H) ;
une soufflante (16) configurée pour entraîner un écoulement d'air à travers un espace intérieur du caisson ;
un échangeur de chaleur (14) situé dans l'espace intérieur du caisson (10H) ;
un dispositif générateur d'ions (190) espacé de l'échangeur de chaleur (14) et couplé à un côté intérieur du caisson (10H) ; et
une commande (C) électriquement reliée à la soufflante (16) et au dispositif générateur d'ions (190), et configurée pour faire fonctionner la soufflante (16) pendant une opération de climatisation et pour faire fonctionner le dispositif générateur d'ions (190) pendant une opération de désinfection ;
dans lequel le dispositif générateur d'ions (190) comporte :
un ioniseur (192) ;
un corps (191b) ayant un espace intérieur (192S) vers lequel l'ioniseur (192) est dirigé, sous la forme d'un corps creux ; et
un ventilateur (18) couplé au corps (191b) et configuré pour entraîner un écoulement d'air passant à travers l'espace intérieur (192S) pour entraîner un écoulement d'un ion généré dans l'ioniseur (192),
dans lequel un élément parmi la soufflante (16) et le dispositif générateur d'ions (190) est configuré pour fonctionner pendant que l'autre élément est arrêté ; et
dans lequel, pendant l'opération de désinfection, la commande (C) est configurée pour régler un temps de fonctionnement du dispositif générateur d'ions (190), sur la base d'informations sur un temps d'exécution de l'opération de climatisation réalisée avant l'opération de désinfection.

2. Climatiseur selon la revendication 1, dans lequel la commande (C) est configurée pour réaliser l'opération de désinfection après la fin de l'opération de climatisation.

3. Climatiseur selon la revendication 1 ou 2, dans lequel, pendant l'opération de désinfection, le temps de fonctionnement du dispositif générateur d'ions (190) devient plus long, lorsque le temps d'exécution devient plus long.

4. Climatiseur selon l'une quelconque des revendications précédentes, dans lequel la commande (C) est configurée pour faire fonctionner le dispositif générateur d'ions (190) pendant un premier temps lorsque le temps d'exécution est inférieur ou égal à un premier temps de référence, pour faire fonctionner le dispositif générateur d'ions (190) pendant un deuxième temps lorsque le temps d'exécution dépasse le premier temps de référence mais est inférieur ou égal à un second temps de référence, et pour faire fonctionner le dispositif générateur d'ions (190) pendant un troisième temps lorsque le temps d'exécution dépasse le second temps de référence,
dans lequel le deuxième temps est supérieur au premier temps, mais inférieur au troisième temps.

5. Climatiseur selon l'une quelconque des revendications précédentes, comportant en outre un capteur (199) situé dans l'espace intérieur du caisson (10H) pour détecter de la poussière dans un air passant à travers le caisson (10H),
dans lequel la commande (C) est configurée pour régler un temps de fonctionnement du dispositif générateur d'ions (190), sur la base d'informations obtenues à partir du capteur (199), pendant l'opération de désinfection.

6. Climatiseur selon la revendication 5, dans lequel le temps de fonctionnement du dispositif générateur d'ions (190) pendant l'opération de désinfection devient plus long lorsqu'une valeur de capteur de poussière fine qui est une quantité de poussière dans l'air passant à travers le caisson (10H), devient plus grande.

7. Climatiseur selon la revendication 5 ou 6, dans lequel, pendant l'opération de désinfection, la commande (C) est configurée pour régler un temps de fonctionnement du dispositif générateur d'ions (190), également sur la base d'informations sur le temps d'exécution de l'opération de climatisation réalisée avant l'opération de désinfection.

8. Climatiseur selon la revendication 6 ou 7, dans lequel la commande (C) est configurée pour calculer une valeur de capteur de poussière fine qui est une quantité de poussière dans l'air passant à travers le caisson (10H), sur la base des informations obtenues à partir du capteur (199),
pour faire fonctionner le dispositif générateur d'ions (190) pendant un premier temps, lorsque le temps d'exécution est inférieur ou égal à un premier temps de référence et que la valeur de capteur de poussière fine est inférieure ou égale à une valeur de référence, et
pour faire fonctionner le dispositif générateur d'ions (190) pendant un deuxième temps, lorsque le temps d'exécution est inférieur ou égal au premier temps de référence et que la valeur de capteur de poussière fine dépasse la valeur de référence,
dans lequel le deuxième temps est supérieur au premier temps.

9. Climatiseur selon la revendication 8, dans lequel la commande (C) est configurée pour faire fonctionner le dispositif générateur d'ions (190) pendant le deuxième temps, lorsque le temps d'exécution dépasse le premier temps de référence et que la valeur de capteur de poussière fine est inférieure ou égale à la valeur de référence, et
pour faire fonctionner le dispositif générateur d'ions (190) pendant le troisième temps, lorsque le temps d'exécution dépasse le premier temps de référence et que la valeur de capteur de poussière fine dépasse la valeur de référence,
dans lequel le troisième temps est supérieur au deuxième temps.

10. Climatiseur selon l'une quelconque des revendications précédentes, dans lequel l'échangeur de chaleur (14) comporte en outre :
un premier échangeur de chaleur (13) ; et
un deuxième échangeur de chaleur (14) situé en aval du premier échangeur de chaleur (13), dans un passage d'air formé par le ventilateur (18),
dans lequel le dispositif générateur d'ions (190) est situé en un centre entre le premier échangeur de chaleur (13) et le second échangeur de chaleur (14).

11. Climatiseur selon la revendication 10, dans lequel l'échangeur de chaleur (150) comporte en outre un troisième échangeur de chaleur (15) situé en aval du deuxième échangeur de chaleur (14), dans le passage d'air formé par le ventilateur (18),
dans lequel le dispositif générateur d'ions (190) comporte :
un premier dispositif générateur d'ions (190a) situé entre le premier échangeur de chaleur (13) et le deuxième échangeur de chaleur (14) ; et
un second dispositif générateur d'ions (190b) situé entre le deuxième échangeur de chaleur (14) et le troisième échangeur de chaleur (15).

12. Climatiseur selon l'une quelconque des revendications précédentes, dans lequel le caisson (10H) comporte une partie supérieure (10T) qui forme un côté supérieur du caisson (10H), et à laquelle le dispositif générateur d'ions (190) est couplé,
dans lequel une extrémité inférieure du dispositif générateur d'ions (190) est située au-dessus d'une extrémité supérieure de l'échangeur de chaleur (13).
